# EUROPEAN PATENT SPECIFICATION

(11) **EP 3 775 274 B1**
(45) Date of publication and mention of the grant of the patent: **20.12.2023**
(21) Application number: 19717477.4
(22) Date of filing: 11.04.2019
(51) Int. Cl.: C12Q 1/6827, C12Q 1/6834, C12Q 1/6886

(54) **DETECTION METHOD OF SOMATIC GENETIC ANOMALIES, COMBINATION OF CAPTURE PROBES AND KIT OF DETECTION**
VERFAHREN ZUM NACHWEIS VON SOMATISCHEN GENETISCHEN ANOMALIEN, KOMBINATION VON FANGSONDEN UND NACHWEISKIT
MÉTHODE DE DÉTECTION D'ANOMALIES GÉNÉTIQUES SOMATIQUES, COMBINAISON DE SONDES DE CAPTURE ET D'UN KIT DE DÉTECTION

(30) Priority: 11.04.2018 EP 18305440
(43) Date of publication of application: 17.02.2021
(73) Proprietor: Université de Bourgogne, 21000 Dijon (FR); CGFL (Centre Georges François Leclerc), 21000 Dijon (FR)
(72) Inventor: BOIDOT, Romain, 21310 ARCEAU (FR); VEGRAN, Frédérique, 21310 ARCEAU (FR); GHIRINGHELLI, François, 21000 DIJON (FR)
(74) Representative: Jacobacci Coralis Harle
(86) International application number: PCT/EP2019/059274
(87) International publication number: WO 2019/197541

(56) References cited:
- WO-A1-00/78945
- WO-A1-2012/092426
- DAVID H. SPENCER ET AL: "Comparison of Clinical Targeted Next-Generation Sequence Data from Formalin-Fixed and Fresh-Frozen Tissue Specimens", JOURNAL OF MOLECULAR DIAGNOSTICS,THE, vol. 15, no. 5, 1 September 2013 (2013-09-01), pages 623-633, XP055479727, US ISSN: 1525-1578, DOI: 10.1016/j.jmoldx.2013.05.004
- ERIC J DUNCAVAGE ET AL: "Targeted next generation sequencing of clinically significant gene mutations and translocations in leukemia", MODERN PATHOLOGY, vol. 25, no. 6, 16 March 2012 (2012-03-16) , pages 795-804, XP055235397, GB ISSN: 0893-3952, DOI: 10.1038/modpathol.2012.29

## Description

### FIELD OF THE INVENTION

The present invention relates to field of cancer detection.

Especially, the present invention relates to a detection method of genetic anomalies potentially present in a tumor using specific capture probes, a kit of detection of these genetic anomalies, and a combination of capture probes.

### DESCRIPTION OF RELATED ART

Cancer is one of the leading causes of death in the world. For instance, in 2010, nearly 1.5 million people (essentially women) developed breast cancer worldwide. It is estimated that 5 to 10% of these cases were hereditary. In addition, more than 1.5 million new cases and more than 0.5 million deaths were reported during 2010 in the United States alone.

Treatment currently performed for cancer is mainly symptomatic therapy that mostly consists of surgical therapy with a combination of radiation therapy and chemotherapy. Owing to advancements in medical technology, cancer is now almost a curable disease if it can be detected early.

Indeed, it is desirable not only to detect cancer, but also to diagnose: a cancer developed in a location invisible to the naked eye, an extent of cancer, a malignancy or postoperative course of cancer, a recurrence, a metastasis, and the like.

The widespread use of Next Generation Sequencing (NGS), also known as massively parallel sequencing, has opened up new avenues for cancer research and diagnosis. NGS will bring huge amounts of new data on cancer, and especially cancer genetics.

NGS represents an effective way to capture a large amount of genomic information about a cancer. Most NGS technologies revolve around sequencing by synthesis. Each DNA fragment to be sequenced is bound to an array, and then DNA polymerase adds labeled nucleotides sequentially. A high-resolution camera captures the signal from each nucleotide becoming integrated and notes the spatial coordinates and time. The sequence at each spot can then be inferred by a computer program to generate a contiguous DNA sequence, referred to as a read.

Multiple technological enhancements have allowed NGS to be more readily implemented in a clinical workflow. Samples now no longer need to be handled differently from standard diagnostic specimens, and recent advances have even enabled increasingly complex genomic data to be derived from a patient's peripheral blood. The concept of precision medicine goes hand in hand with an understanding of the cancer genome as determined by NGS.

Before the development of NGS, tumor genotyping was performed only on specific genomic loci that were known to be frequently mutated in cancer, which are known as "hotspots", in general by Sanger sequencing. These approaches were best suited to recurrent activating mutations in oncogenes, such as in the *KRAS* gene in colon and lung cancer.

However, these approaches were insufficient to identify alterations in tumor suppressors (in which an alteration anywhere in the gene may impact its function) or the increasingly complex area of "long tail" hotspot alterations in oncogenes. In addition, these approaches only allow a very limited exploration of the human genome.

Thus, current assay options involve approaches that may capture known cancer genes ("gene panels"), whole-exome, whole-genome and/or whole-transcriptome approaches. There are several trade-offs to increasing the portion of the genome that is sequenced. The first is a loss of coverage for the same amount of sequencing. Coverage, or depth, is defined as the average number of mappable reads at a given locus in your panel. Lower coverage limits the ability to confidently call a variant of low allele fraction to be biologically real and not a technical artifact. A second is that whole-genome and whole-exome sequencing require germline sequencing to enhance the identification of true somatic variants, which may uncover incidental clinically relevant inherited disorders.

To achieve these last approaches, the large majority of the cancer detection kits on the market are based on amplicon sequencing.

Amplicon sequencing enriches target genes by PCR with a set of primers for exons of selected genes prior to NGS analysis. Especially, the set of primers will amplify different and large DNA fragments. These protocols have the advantage of less required input DNA and less turnaround time than hybrid capture methods, which is critical for clinical application, but potentially PCR amplification can bias the observed allele fraction. It also pulls information out of a lower percentage of starting material, further increasing the chance of bias in calling copy number variations. The informatics analysis is relatively easy, as any read that does not map to a locus between primers can be disregarded.

A downside of this simplicity is that the assay is inherently unable to detect unexpected fusions, because either the 5' or 3' primer would fail to bind the translocated DNA. Also, this sequencing method requires DNA which is in a perfect state. In addition, the specific primers of these different fragments require similar hybridization and elongation conditions. These conditions being difficult to meet, the amplicon sequencing generates a large amount of artefacts.

DAVID H. SPENCER ET AL, THE JOURNAL OF MOLECULAR DIAGNOSTICS, vol. 15, no. 5, 1 September 2013 (2013-09-01), pages 623-633, relates to comparison of clinical targeted Next-Generation Sequence data from formalin-fixed and fresh-frozen tissue specimens. WO2012/092426A1 relates to optimization of multigene analysis of tumor samples.

Hence, there is a need to develop a new detection method of somatic genetic anomalies present in a tumor which enables to detect various types of cancer with high sensitivity at one time, and preferably for different patients.

Especially, there is a need to develop a new detection method which allows to provide a personalized medicine for each patient.

### SUMMARY OF THE INVENTION

The present invention meets these needs since the inventors, surprisingly and unexpectedly, discovered that a specific combination of capture probes embedded in hybrid capture enables to obtain a high sensitive measurement method for detecting somatic gene anomalies and this, for various type of cancer and at one time.

Moreover, the inventors have discovered that the specific combination of capture probes embedded in hybrid capture is capable of not only detecting cancer, but also diagnosing cancer having developed in a location invisible to the naked eye, the extent of cancer, the malignancy or postoperative course of cancer, recurrence, metastasis, and the like.

The present invention thus provides a detection method of somatic genetic anomalies potentially present in a solid tissue sample (potentially cancerous), taken from a subject, wherein said detection method comprises the following steps:
(a) shearing of a genomic DNA extracted from said tissue sample, to obtain sheared genomic DNA;
(b) preparing a library by hybridization capture from the sheared genomic DNA obtained at step (a), by the implementation of a combination at least 51 capture probes having at least 110 nucleotides in length and at least 75 % sequence homology with respectively SEQ ID NO:1 to SEQ ID NO: 51, said capture probes being able to hybridize on fragments from the following target regions from : ALK, BRAF, EGFR, EIF1AY, ERBB2, ERBB4, KDR, KIT, KRAS, MAP2K1, MET, NRAS, PDGFRA, SLC28A1, TP53 ;
(c) sequencing of sheared genomic DNA contained in the library issued at step (b), and
(d) analyzing of the sequences of said sheared genomic DNA obtained at step (c), for detecting somatic genetic anomalies in said tissue sample.

The present invention enables a high sensitivity of the analysis of 15, full coding sequence (and closed intron sequences) genes with a high reliability. This is the consequence of a very low background signal, a specific design targeting theranostics studied genes, and the use of internal controls. Finally, this design can be used on solid (tumor) and liquid (blood) samples.

The present invention concerns a combination of capture probes, comprising at least 51 capture probes comprising at least 110 nucleotides and at least 75 % sequence homology with respectively the probes of SEQ ID NO:1 to SEQ ID NO:51.

Preferably, the combination of capture probes comprises further at least 100 additional capture probes, preferably at least 200, in particular at least 300, and ideally at least 1178 additional capture probes comprising at least 110 nucleotides and at least 75 % sequence homology with respectively the probes of SEQ ID NO:52 to SEQ ID NO:1229.

The present invention also refers to a kit of detection of somatic genetic anomalies potentially present in a tissue sample potentially cancerous taken from a subject, comprising the combination of capture probes as defined above.

The various embodiments of the present invention are especially described in the detailed specification hereafter. These embodiments may be considered separately or be combined with each other.

### DETAILED DESCRIPTION OF THE INVENTION AND PREFERRED EMBODIMENTS

The practice of the present invention will employ, unless otherwise indicated, conventional techniques of molecular biology, microbiology and recombinant DNA techniques, which are within the skill of the art. Such techniques are explained fully in the literature. See, e.g., Sambrook, Fritsch & Maniatis, Molecular Cloning: A Laboratory Manual, Fourth Edition (2012); Oligonucleotide Synthesis (M. J. Gait, ed., 1984); Nucleic Acid Hybridization (B. D. Hames & S. J. Higgins, eds., 1984); A Practical Guide to Molecular Cloning (B. Perbal, 1984); and a series, Methods in Enzymology (Academic Press, Inc.).

### A -DEFINITIONS

The terms "comprise" (and any grammatical variation thereof, such as "comprises" and "comprising"), "have" (and any grammatical variation thereof, such as "has" and "having"), "contain" (and any grammatical variation thereof, such as "contains" and "containing"), and "include" (and any grammatical variation thereof, such as "includes" and "including") are openended linking verbs. They are used to specify the presence of stated features, integers, steps or components or groups thereof, but do not preclude the presence or addition of one or more other features, integers, steps or components or groups thereof. As a result, a method, or a step in a method, that "comprises," "has," "contains," or "includes" one or more steps or elements possesses those one or more steps or elements, but is not limited to possessing only those one or more steps or elements.

Unless otherwise indicated, all numbers or expressions referring to quantities of ingredients, ranges, reaction conditions, etc. used herein are to be understood as modified in all instances by the term "about."

Also unless otherwise indicated, the indication of an interval of values « from X to Y » or "between X to Y", according to the present invention, means as including the values of X and Y.

As used in the specification and claims, the singular forms "a", "an" and "the" can include plural references unless the context clearly dictates otherwise. For example, the term "a cell" can include a plurality of cells, including mixtures thereof.

The cancer is the class of disease characterized by rapid and uncontrolled proliferation of cells within a tissue of a multi-tissue eukaryote. Cancers are generally thought to be genetic diseases of somatic cells, arising through sequential mutations that create oncogenes and inactivate tumor-suppressor genes.

The term "subject", as used herein, generally refers to a biological entity containing expressed genetic materials. The biological entity can be a plant, animal, or microorganism, including, e.g., bacteria, viruses, fungi, and protozoa. The subject can be tissues, cells and their progeny of a biological entity obtained *in vivo* or cultured *in vitro.* The subject can be a mammal. The mammal can be a human. The human may be diagnosed or suspected of being at high risk for a disease. The disease can be cancer. The human may not be diagnosed or suspected of being at high risk for a disease.

As used herein, a "sample" or "nucleic acid sample" can refer to any substance containing or presumed to contain nucleic acid. The sample can be a biological sample obtained from a subject. The nucleic acids can be RNA, DNA, e.g., genomic DNA, mitochondrial DNA, viral DNA, synthetic DNA, or cDNA reverse transcribed from RNA.

The term "genomic DNA" consists advantageously in DNA from the genome containing all coding (exon) and noncoding (intron and other) sequences.

The nucleic acids in a nucleic acid sample generally serve as templates for extension of a hybridized primer. In some embodiments, the biological sample is a liquid sample. The liquid sample can be whole blood, plasma, serum, ascites, cerebrospinal fluid, sweat, urine, tears, saliva, buccal sample, cavity rinse, or organ rinse. The liquid sample can be an essentially cell-free liquid sample (e.g., plasma, serum, sweat, plasma, urine, sweat, tears, saliva, sputum, cerebrospinal fluid). In other embodiments, the biological sample is a solid biological sample, e.g., feces or tissue biopsy, e.g., a tumor biopsy. A sample can also comprise *in vitro* cell culture constituents (including but not limited to conditioned medium resulting from the growth of cells in cell culture medium, recombinant cells and cell components). The sample can comprise a single cell, e.g., a cancer cell, a circulating tumor cell, a cancer stem cell, and the like.

"Nucleotides" and "nt" are used interchangeably herein to generally refer to biological molecules that can form nucleic acids. Nucleotides can have moieties that contain not only the known purine and pyrimidine bases, but also other heterocyclic bases that have been modified. Such modifications include methylated purines or pyrimidines, acylated purines or pyrimidines, alkylated riboses, or other heterocycles. In addition, the term "Nucleotide" includes those moieties that contain hapten, biotin, or fluorescent labels and may contain not only conventional ribose and deoxyribose sugars, but other sugars as well.

Modified nucleosides or nucleotides also include modifications on the sugar moiety, e.g., wherein one or more of the hydroxyl groups are replaced with halogen atoms or aliphatic groups, are functionalized as ethers, amines, or the like. Modified nucleosides or nucleotides can also include peptide nucleic acid (PNA). Peptide nucleic acid generally refers to oligonucleotides in which the deoxyribose backbone has been replaced with a backbone having peptide linkages. Each subunit generally has attached a naturally occurring or non-naturally occurring base. One exemplary PNA backbone is constructed of repeating units of N-(2-aminoethyl)glycine linked through amide bonds. PNA can bind both DNA and RNA to form PNA/DNA or PNA/RNA duplexes. The resulting PNA/DNA or PNA/RNA duplexes can be bound with greater affinity than corresponding DNA/DNA or DNA/RNA duplexes as evidence by their higher melting temperatures (Tm). The neutral backbone of the PNA also can render the Tm of PNA/DNA(RNA) duplexes to be largely independent of salt concentration in a reaction mixture. Thus the PNA/DNA duplex can offer an advantage over DNA/DNA duplex interactions which are highly dependent on ionic strength. Exemplary embodiments of PNA are described in US Patent Nos. 7,223,833 and 5,539,083.

"Nucleotides" can also include nucleotides comprising a Tm-enhancing base (e.g., a Tm-base enhancing nucleotide). Exemplary Tm-enhancing base nucleotides include, but are not limited to nucleotides with Superbases^{™}, locked nucleic acids (LNA) or bridged nucleic acids (BNA). BNA and LNA generally refer to modified ribonucleotides wherein the ribose moiety is modified with a bridge connecting the 2' oxygen and 4' carbon. Generally, the bridge "locks" the ribose in the 3'-endo (North) conformation, which is often found in the A- form duplexes. The term "locked nucleic acid" (LNA) generally refers to a class of BNAs, where the ribose ring is "locked" with a methylene bridge connecting the 2'-0 atom with the 4'-C atom. LNA nucleosides containing the six common nucleobases (T, C, G, A, U and mC) that appear in DNA and RNA are able to form base-pairs with their complementary nucleosides according to the standard Watson-Crick base pairing rules. Accordingly, Tm- enhancing base nucleotides such as BNA and LNA nucleotides can be mixed with DNA or RNA bases in an oligonucleotide whenever desired. The locked ribose conformation enhances base stacking and backbone pre-organization. Base stacking and backbone pre- organization can give rise to an increased thermal stability (e.g., increased Tm) and discriminative power of duplexes. LNA can discriminate single base mismatches under conditions not possible with other nucleic acids. Locked nucleic acid is disclosed for example in WO 99/14226.. Nucleotides can also include modified nucleotides as described in European Patent Application No. EP1995330.

Other modified nucleotides can include 5-Me-dC-CE phosphoramidite, 5-Me-dC- CPG, 2-Amino-dA-CE phosphoramidite, N4-Et-dC-CE Phosphoramidite, N4-Ac-N4-Et-dC- CE Phosphoramidite, N6-Me-dA-CE Phosphoramidite, N6-Ac-N6-Me-dA-CE

Phosphoramidite, Zip nucleic acids (ZNA^{®}, described in the literature), 5'-Trimethoxystilbene Cap Phosphoramidite, 5'-Pyrene Cap Phosphoramidite, 3'-Uaq Cap CPG. (Glen Research).

Yet other modified nucleotides can include nucleotides with modified nucleoside bases such as, e.g., 2-Aminopurine, 2,6-Diaminopurine, 5-Bromo-deoxyuridine, deoxyuridine, Inverted dT, inverted ddT, ddC, 5-Methyl deoxyCytidine, deoxylnosine, 5-Nitroindole, 2'-0-Methyl RNA bases, Hydroxmethyl dC, Iso-dG and Iso-dC (Eragen Biosciences, Inc), 2'-Fluoro bases having a fluorine modified ribose.

The terms "polynucleotides", "nucleic acid", "nucleotides" and "oligonucleotides" can be used interchangeably. They can refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers.

A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

The term "target polynucleotide," "target region", or "target", as used herein, generally refers to a polynucleotide of interest under study. In certain embodiments, a target polynucleotide contains one or more sequences that are of interest and under study. A target polynucleotide can comprise, for example, a genomic sequence. The target polynucleotide can comprise a target sequence whose presence, amount, and/or nucleotide sequence, or changes in these, are desired to be determined.

The target polynucleotide can be a region of gene associated with a disease. In general, the region is an exon. In some embodiments, the gene is a druggable target. The term "druggable target", as used herein, generally refers to a gene or cellular pathway that is modulated by a disease therapy. The disease can be cancer. Accordingly, the gene can be a known cancer-related gene. Especially, the cancer-related gene is selected from the group consisting of ALK, BRAF, EGFR, EIF1AY, ERBB2, ERBB4, KDR, KIT, KRAS, MAP2K1, MET, NRAS, PDGFRA, SLC28A1, TP53.

The term "genomic sequence", as used herein, generally refers to a sequence that occurs in a genome. Because RNAs are transcribed from a genome, this term encompasses sequences that exist in the nuclear genome of an organism, as well as sequences that are present in a cDNA copy of an RNA (e.g., an mRNA) transcribed from such a genome.

The terms "anneal", "hybridize" or "bind," can refer to two polynucleotide sequences, segments or strands, can be used interchangeably and have the usual meaning in the art. Two complementary sequences (e.g., DNA and/or RNA) can anneal or hybridize by forming hydrogen bonds with complementary bases to produce a double-stranded polynucleotide or a double-stranded region of a polynucleotide.

As used herein, the term "complementary" generally refers to a relationship between two antiparallel nucleic acid sequences in which the sequences are related by the base-pairing rules: A pairs with T or U and C pairs with G. A first sequence or segment that is "perfectly complementary" to a second sequence or segment is complementary across its entire length and has no mismatches. A first sequence or segment is "substantially complementary" to a second sequence of segment when a polynucleotide consisting of the first sequence is sufficiently complementary to specifically hybridize to a polynucleotide consisting of the second sequence.

The term "duplex," or "duplexed," as used herein, can describe two complementary polynucleotides that are base-paired, e.g., hybridized together.

As used herein, the term "Tm" generally refers to the melting temperature of an oligonucleotide duplex at which half of the duplexes remains hybridized and half of the duplexes dissociates into single strands. See Sambrook and Russell (2001; Molecular Cloning: A Laboratory Manual, 3rd ed., Cold Spring Harbor Press, Cold Spring Harbor N.Y., ch. 10).

As used herein, "amplification" of a nucleic acid sequence generally refers to *in vitro* techniques for enzymatically increasing the number of copies of a target sequence. Amplification methods include both asymmetric methods (in which the predominant product is single-stranded) and conventional methods (in which the predominant product is double-stranded). A "round" or "cycle" of amplification can refer to a PCR cycle in which a double stranded template DNA molecule is denatured into single-stranded templates, forward and reverse primers are hybridized to the single stranded templates to form primer/template duplexes, primers are extended by a DNA polymerase from the primer/template duplexes to form extension products. In subsequent rounds of amplification the extension products are denatured into single stranded templates and the cycle is repeated.

The terms "template", "template strand", "template DNA" and "template nucleic acid" can be used interchangeably herein to refer to a strand of DNA that is copied by an amplification cycle.

The term "denaturing," as used herein, generally refers to the separation of a nucleic acid duplex into two single strands.

The term "extending", as used herein, generally refers to the extension of a primer hybridized to a template nucleic acid by the addition of nucleotides using an enzyme, e.g., a polymerase.

A "primer "is generally a nucleotide sequence (e.g., an oligonucleotide), generally with a free 3'-OH group, that hybridizes with a template sequence (such as a target polynucleotide, or a primer extension product) and is capable of promoting polymerization of a polynucleotide complementary to the template. A primer can be, for example, a sequence of the template (such as a primer extension product or a fragment of the template created following Rase cleavage of a template -DNA complex) that is hybridized to a sequence in the template itself (for example, as a hairpin loop), and that is capable of promoting nucleotide polymerization. Thus, a primer can be an exogenous (e.g., added) primer or an endogenous (e.g., template fragment) primer.

The terms "determining", "measuring", "evaluating", "assessing", "assaying", and "analyzing" can be used interchangeably herein to refer to any form of measurement, and include determining if an element is present or not. These terms can include both quantitative and/or qualitative determinations. Assessing may be relative or absolute. "Assessing the presence of" can include determining the amount of something present, as well as determining whether it is present or absent.

The term "free in solution," as used here, can describe a molecule, such as a polynucleotide, that is not bound or tethered to a solid support.

The term "genomic fragment", as used herein, can refer to a region of a genome, e.g., an animal or plant genome such as the genome of a human, monkey, rat, fish or insect or plant. A genomic fragment may or may not be adaptor ligated. A genomic fragment may be adaptor ligated (in which case it has an adaptor ligated to one or both ends of the fragment, to at least the 5' end of a molecule), or non-adaptor ligated.

"Pre-amplification", as used herein, generally refers to non-clonal amplification of nucleic acids. For example, pre-amplification of a nucleic acid library is generally performed prior to clonal amplification of the library and/or loading onto a sequencer.

The term "ligase", as used herein, generally refers to an enzyme that is commonly used to join polynucleotides together or to join the ends of a single polynucleotide.

The term "ligation", as used herein, generally refers to the joining of two ends of polynucleotides or the joining of ends of a single polynucleotide by the formation of a covalent bond between the ends to be joined. The covalent bond can be a phosphodiester bond.

The term "ATP-dependent ligation", as used herein, generally refers to ligation by an ATP-dependent ligase. An exemplary mechanism of ATP-dependent ligation is described herein.

"Donor" and "acceptor" nucleic acid species generally refer to two distinct populations of nucleic acid molecules to be joined in a ligation reaction. The "donor" species generally refers to a population of nucleic acid molecules which may accept a nucleoside monophosphate (NMP) at either a 5' or 3' end. The "acceptor" species generally refers to a second population of nucleic acid molecules containing a 3 Or 5' OH group which may be ligated to the "donor" species via the NMP at either the 5' or 3' end of the donor species.

The donor and acceptor species can be any nucleic acid species. They can be, for example, polynucleotides isolated from a biological source. The biological source can be a subject. Exemplary biological sources and subjects are described herein. They can be oligonucleotides. Methods for preparing oligonucleotides of specific sequence are known in the art, and include, for example, cloning and restriction of appropriate sequences and direct chemical synthesis. Chemical synthesis methods may include, for example, the phosphotriester method described by Narang et al, 1979, Methods in Enzymology 68:90, the phosphodiester method disclosed by Brown et al, 1979, Methods in Enzymology 68: 109, the diethylphosphoramidate method disclosed in Beaucage et al, 1981, Tetrahedron Letters 22: 1859, and the solid support method disclosed in U.S. Pat. No. 4,458,066. They can be RNA or DNA. The DNA can be partially or fully denatured DNA. The DNA can be single stranded (ss) DNA. Partially denatured can be "frayed" at ends such that a "frayed" end can comprise 1, 2, 3, 4, 5, or more than 5 non-annealed nucleotides.

The donor and/or acceptor nucleic acid species can be of any size, ranging from, e.g., 1-50 nt, 10-100 nt, 50-200 nt, 100-400 nt, 200-600 nt, 500-1000 nt, 800-2000 nt, or greater than 2000 nt. In some embodiments, the donor and/or acceptor nucleic acid species is over 120 nt long.

The donor or acceptor nucleic acid species can include, e.g., genomic nucleic acids, adaptor sequences, and/or barcode sequences. The donor or acceptor nucleic acid species can include oligonucleotides. The donor or acceptor nucleic acid species can comprise a detectable label or affinity tag.

The detectable label can be any molecule that enables detection of a molecule to be detected. Non-limiting examples of detectable labels include, e.g., chelators, photoactive agents, radioactive moieties (e.g., alpha, beta and gamma emitters), fluorescent agents, luminescent agents, paramagnetic ions, or enzymes that produce a detectable signal in the presence of certain reagents (e.g., horseradish peroxidase, alkaline phosphatase, glucose oxidase).

Exemplary fluorescent compounds include, e.g., fluorescein isothiocyanate, rhodamine, phycoerytherin, phycocyanin, allophycocyanin, o-phthaldehyde, fluorescamine, and commercially available fluorophores such as Alexa Fluor 350, Alexa Fluor 488, Alexa Fluor 532, Alexa Fluor 546, Alexa Fluor 568, Alexa Fluor 594, Alexa Fluor 647, DyLight dyes such as DyLight 488, DyLight 594, DyLight 647, and BODIPY dyes such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine and Texas Red. Such compounds are commercially available (see, e.g., Molecular Probes, Inc.).

The affinity tag can be selected to have affinity to a capture moiety. The affinity tag can comprise, by way of non-limiting example only, biotin, desthiobiotin, histidine, polyhistidin, myc, hemagglutinin (HA), FLAG, a fluorescence tag, a tandem affinity purification (TAP) tag, a glutathione S transferase (GST) tag, or derivatives thereof. The capture moiety can comprise, e.g., avidin, streptavidin, Neutravidin^{™}, nickel, or glutathione or other molecule capable of binding the affinity tag.

In some embodiments, the acceptor species and the donor species can be the same species. For example, in some embodiments a user may desire to circularize a linear nucleic acid or to form concatemers of a single nucleic acid species.

The term "reaction mixture" as used herein generally refers to a mixture of components necessary to effect a desired reaction. The mixture may further comprise a buffer (e.g., a Tris buffer). The reaction mixture may further comprise a monovalent salt. The reaction mixture may further comprise a cation, e.g., Mg and/or Mn. The concentration of each component is well known in the art and can be further optimized by an ordinary skilled artisan. In some embodiments, the reaction mixture also comprises additives including, but not limited to, non-specific background/blocking nucleic acids (e.g., salmon sperm DNA), non-specific background/blocking proteins (e.g., bovine serum albumin, non-fat dry milk) biopreservatives (e.g. sodium azide), PCR enhancers (e.g. Betaine, Trehalose, etc.), and inhibitors (e.g. RNAse inhibitors). In some embodiments, a nucleic acid sample is admixed with the reaction mixture.

A "primer binding site" can refer to a site to which a primer hybridizes in an oligonucleotide or a complementary strand thereof.

The term "separating", as used herein, can refer to physical separation of two elements (e.g., by size, affinity, degradation of one element etc.).

The term "sequencing", as used herein, can refer to a method by which the identity of at least 10 consecutive nucleotides (e.g., the identity of at least 20, at least 50, at least 100, at least 200, or at least 500 or more consecutive nucleotides) of a polynucleotide are obtained.

The term "adaptor-ligated", as used herein, can refer to a nucleic acid that has been ligated to an adaptor. The adaptor can be ligated to a 5' end or a 3' end of a nucleic acid molecule, or can be added to an internal region of a nucleic acid molecule.

The term "bridge PCR" can refer to a solid-phase polymerase chain reaction in which the primers that are extended in the reaction are tethered to a substrate by their 5' ends. During amplification, the amplicons form a bridge between the tethered primers. Bridge PCR (which may also be referred to as "cluster PCR") is used in Illumina's Solexa platform. Bridge PCR and Illumina's Solexa platform are generally described in a variety of publications, e.g., Gudmundsson et al (Nat. Genet. 2009 41 : 1122-6), Out et al (Hum. Mutat. 2009 30: 1703-12) and Turner (Nat. Methods 2009 6:315-6), U.S. Pat. No. 7,115,400, and publication application publication nos. US20080160580 and US20080286795.

The term "barcode sequence" as used herein, generally refers to a unique sequence of nucleotides that can encode information about an assay. A barcode sequence can encode information relating to the identity of an interrogated allele, identity of a target polynucleotide or genomic locus, identity of a sample, a subject, or any combination thereof. A barcode sequence can be a portion of a primer, a reporter probe, or both. A barcode sequence may be at the 5'-end or 3'-end of an oligonucleotide, or may be located in any region of the oligonucleotide. A barcode sequence may or may not be part of a template sequence. Barcode sequences may vary widely in size and composition; the following references provide guidance for selecting sets of barcode sequences appropriate for particular embodiments: Brenner, U.S. Pat. No. 5,635,400; Brenner et al, Proc. Natl. Acad. Sci., 97: 1665-1670 (2000); Shoemaker et al, Nature Genetics, 14: 450-456 (1996); Morris et al, European patent publication 0799897A1; Wallace, U.S. Pat. No. 5,981,179. A barcode sequence may have a length of about 4 to 36 nucleotides, about 6 to 30 nucleotides, or about 8 to 20 nucleotides.

The terms "somatic genetic anomalies" relate to an alteration or mutation in DNA that occurs after conception. Somatic mutations can occur in any of the cells of the body except the germ cells (sperm and egg) and therefore are not passed on to children. These alterations can cause cancer or other diseases.

The term "mutation", as used herein, generally refers to a change of the nucleotide sequence of a genome. Mutations can involve large sections of DNA (e.g., copy number variation). Mutations can involve whole chromosomes (e.g., aneuploidy). Mutations can involve small sections of DNA. Examples of mutations involving small sections of DNA include, e.g., point mutations or single nucleotide polymorphisms, multiple nucleotide polymorphisms, insertions (e.g., insertion of one or more nucleotides at a locus), multiple nucleotide changes, deletions (e.g., deletion of one or more nucleotides at a locus), and inversions (e.g., reversal of a sequence of one or more nucleotides).

The term "locus", as used herein, can refer to a location of a gene, nucleotide, or sequence on a chromosome. An "allele" of a locus, as used herein, can refer to an alternative form of a nucleotide or sequence at the locus. A "wild-type allele" generally refers to an allele that has the highest frequency in a population of subjects. A "wild-type" allele generally is not associated with a disease. A "mutant allele" generally refers to an allele that has a lower frequency than a "wild-type allele" in the general population and may be associated with a disease. A "mutant allele" may not have to be associated with a disease. The term "interrogated allele" generally refers to the allele that an assay is designed to detect.

The term "single nucleotide polymorphism", or "SNP", as used herein, generally refers to a type of genomic sequence variation resulting from a single nucleotide substitution within a sequence. "SNP alleles" or "alleles of a SNP" generally refer to alternative forms of the SNP at particular locus. The term "interrogated SNP allele" generally refers to the SNP allele that an assay is designed to detect.

The term "copy number variation" or "CNV" refers to differences in the copy number of genetic information. In many aspects it refers to differences in the per genome copy number of a genomic region. For example, in a diploid organism the expected copy number for autosomal genomic regions is 2 copies per genome. Such genomic regions should be present at 2 copies per cell. For a recent review see Zhang et al. Annu. Rev. Genomics Hum. Genet. 2009. 10:451 - 81. CNV is a source of genetic diversity in humans and can be associated with complex disorders and disease, for example, by altering gene dosage, gene disruption, or gene fusion. They can also represent benign polymorphic variants. CNVs can be large, for example, larger than 1 Mb, but many are smaller, for example between 100 bases and 1 Mb. More than 38,000 CNVs greater than 100 bases (and less than 3 Mb) have been reported in humans. Along with SNPs these CNVs account for a significant amount of phenotypic variation between individuals. In addition to having deleterious impacts, e.g. causing disease, they may also result in advantageous variation.

In certain cases, an oligonucleotide used in the method described herein may be designed using a reference genomic region, i.e., a genomic region of known nucleotide sequence, e.g., a chromosomal region whose sequence is deposited at NCBI's Genbank database or other database, for example.

The term "genotyping", as used herein, generally refers to a process of determining differences in the genetic make-up (genotype) of an individual by examining the individual's DNA sequence using biological assays and comparing it to another individual's sequence or a reference sequence.

A "plurality" generally contains at least 2 members. In certain cases, a plurality may have at least 10, at least 100, at least 100, at least 10,000, at least 100,000, at least 1000000, at least 10000000, at least 100000000, or at least 1000000000 or more members.

The term "separating", as used herein, generally refers to physical separation of two elements (e.g., by cleavage, hydrolysis, or degradation of one of the two elements).

The terms "label" and "detectable moiety" can be used interchangeably herein to refer to any atom or molecule which can be used to provide a detectable signal, and which can be attached to a nucleic acid or protein. Labels may provide signals detectable by fluorescence, radioactivity, colorimetry, gravimetry, X-ray diffraction or absorption, magnetism, enzymatic activity, and the like.

### B- METHOD OF DETECTION

Aspects of the invention relate to a method, a combination of capture probes and kits comprising said combination of capture probes that improve the monitoring and treatment of a subject suffering from a disease, especially cancer.

The cancer can be a tumor, a leukemia such as acute leukemia, acute t-cell leukemia, acute lymphocytic leukemia, acute myelocytic leukemia, myeloblastic leukemia, promyelocytic leukemia, myelomonocytic leukemia, monocytic leukemia, erythroleukemia, chronic leukemia, chronic myelocytic (granulocytic) leukemia, or chronic lymphocytic leukemia, polycythemia vera, lymphomas such as Hodgkin's lymphoma, follicular lymphoma or non-Hodgkin's lymphoma, multiple myeloma, Waldenstrom's macroglobulinemia, heavy chain disease, solid tumors, sarcomas, carcinomas such as, e.g., fibrosarcoma, myxosarcoma, liposarcoma, chondrosarcoma, osteogenic sarcoma, lymphangio sarcoma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic, carcinoma, renal cell carcinoma, hepatoma, bile duct carcinoma, choriocarcinoma, seminoma, embryonal carcinoma, Wilms' tumor, cervical cancer, uterine cancer, testicular tumor, lung carcinoma, small cell lung carcinoma, bladder carcinoma, epithelial carcinoma, glioma, craniopharyngioma, ependymoma, pinealoma, hemangioblastoma, acoustic neuroma, oligodendroglioma, meningioma, melanoma, neuroblastoma, retinoblastoma, endometrial cancer, non-small cell lung cancer.

The subject can be suspected to or known to harbor a solid tumor, or can be a subject who previously harbored a solid tumor.

Especially, the present invention relates to a detection method of somatic genetic anomalies potentially present in a tissue sample, taken from a subject, wherein said detection method comprises the following steps:
(a) shearing of a genomic DNA extracted from said tissue sample, to obtain sheared genomic DNA;
(b) preparing a library by hybridization capture from the sheared genomic DNA obtained at step (a), by the implementation of at least 51 capture probes having at least 110 nucleotides in length and at least 75 % sequence homology with respectively SEQ ID NO:1 to SEQ ID NO:51, said capture probes being able to hybridize on fragments from the following target regions: ALK, BRAF, EGFR, EIF1AY, ERBB2, ERBB4, KDR, KIT, KRAS, MAP2K1, MET, NRAS, PDGFRA, SLC28A1, TP53 ;
(c) sequencing of sheared genomic DNA from the library issued at step (b), and
(d) analyzing of the sequences of said sheared genomic DNA obtained at step (c), for detecting somatic genetic anomalies in said tissue sample that may be cancerous.

Several embodiments of the sequencing methods and assays for determining mutational status of a sample are described herein.

### Sample

According to the invention, the starting sample can be a biological sample obtained from a subject. The biological samples may be a tumor sample and normal cells from the subject. In particular embodiments, the sample is a solid biological sample, e.g., a tumor sample.

In general, nucleic acid is isolated from the tumor sample and normal cells using any methods known in the art. The nucleic acid is DNA, and in particular genomic DNA..

The genomic DNA from the tumor sample and normal cells can be used to prepare a subject-specific tumor genomic DNA library and/or normal genomic DNA library. DNA libraries (and in particular genomic DNA library) can be used for sequencing by a sequencing platform

In some embodiments, the solid biological sample is processed prior to the probe-based assay. Processing can comprise fixation in a formalin solution, followed by embedding in paraffin (e.g., is a FFPE sample). Processing can alternatively comprise freezing of the sample prior to conducting the probe- based assay. In some embodiments, the sample is neither fixed nor frozen. The unfixed, unfrozen sample can be, by way of example only, stored in a storage solution configured for the preservation of nucleic acid. Exemplary storage solutions are described herein. In some embodiments, non-nucleic acid materials can be removed from the starting material using enzymatic treatments (for example, with a protease).

The sample can optionally be subjected to homogenization, sonication, French press, dounce, freeze/thaw, which can be followed by centrifugation. The centrifugation may separate nucleic acid-containing fractions from non- nucleic acid-containing fractions.

In some embodiments, the sample is a liquid biological sample. Exemplary liquid biological samples are described herein. In some embodiments, the liquid biological sample is a blood sample (e.g., whole blood, plasma, or serum). In some embodiments, a whole blood sample is subjected to acellular components (e.g., plasma, serum) and cellular components by use of a Ficoll reagent described in detail Fuss et al, Curr Protoc Immunol (2009) Chapter 7:Unit7.1,

Nucleic acid can be isolated from the biological sample using any means known in the art. For example, nucleic acid can be extracted from the biological sample using liquid extraction (e.g, Trizol, DNAzol) techniques. Nucleic acid can also be extracted using commercially available kits (e.g., Qiagen DNeasy kit, QIAamp kit, Qiagen Midi kit, QIAprep spin kit, Promega Maxwell kits...).

### Next generation sequencing platforms

The detection method of the invention comprises a sequencing step of sheared genomic DNA contained in a library obtained by hybridization capture with a specific combination of capture probes.

The sequencing platform can be a next-generation sequencing (NGS) platform. In some embodiments, the method further comprises sequencing the nucleic acid libraries using NGS technology. NGS technology can involve sequencing of clonally amplified DNA templates or single DNA molecules in a massively parallel fashion (e.g. as described in Volkerding et al. Clin Chem 55:641-658 [2009]; Metzker M Nature Rev 1 1:31-46 [2010]). In addition to high-throughput sequence information, NGS provides digital quantitative information, in that each sequence read is a countable "sequence tag" representing an individual clonal DNA template or a single DNA molecule.

The next-generation sequencing platform can be a commercially available platform. Commercially available platforms include, e.g., platforms for sequencing-by-synthesis, ion semiconductor sequencing, pyrosequencing, reversible dye terminator sequencing, sequencing by ligation, single-molecule sequencing, sequencing by hybridization, and nanopore sequencing. Platforms for sequencing by synthesis are available from, e.g., Illumina, 454 Life Sciences, Helicos Biosciences, and Qiagen. Illumina platforms can include, e.g., Illumina's Solexa platform, Illumina's Genome Analyzer, MiSeq, NextSeq500, Novaseq, HiSeq and are described in Gudmundsson et al (Nat. Genet. 2009 41 : 1122-6), Out et al (Hum. Mutat. 2009 30: 1703- 12) and Turner (Nat. Methods 2009 6:315-6), U.S. Patent Application Pub nos. US20080160580 and US20080286795, U.S. Patent Nos. 6306597, 7115400, and 7232656. 454 Life Science platforms include, e.g., the GS Flex and GS Junior, and are described in U.S. Patent No. 7,323,305. Platforms from Helicos Biosciences include the True Single Molecule Sequencing platform. Platforms for ion semiconductor sequencing include, e.g., the Ion Torrent Personal Genome Machine (PGM) and are described in U.S. Patent No. 7948015. Platforms for pryosequencing include the GS Flex 454 system and are described in U.S. Patent Nos. 7211390; 7244559; 7264929. Platforms and methods for sequencing by ligation include, e.g., the SOLiD sequencing platform and are described in U.S. Patent No. 5750341. Platforms for single-molecule sequencing include the SMRT system from Pacific Bioscience and the Helicos True Single Molecule Sequencing platform.

For instance, the DNA sequencing technology can utilize the Ion Torrent sequencing platform, which pairs semiconductor technology with a sequencing chemistry to directly translate chemically encoded information (A, C, G, T) into digital information (0, 1) on a semiconductor chip. Without wishing to be bound by theory, when a nucleotide is incorporated into a strand of DNA by a polymerase, a hydrogen ion is released as a byproduct. The Ion Torrent platform detects the release of the hydrogen atom as a change in pH. A detected change in pH can be used to indicate nucleotide incorporation. The Ion Torrent platform comprises a high-density array of micro-machined wells to perform this biochemical process in a massively parallel way. Each well holds a different library member, which may be clonally amplified. Beneath the wells is an ion-sensitive layer and beneath that an ion sensor. The platform sequentially floods the array with one nucleotide after another. When a nucleotide, for example a C, is added to a DNA template and is then incorporated into a strand of DNA, a hydrogen ion will be released. The charge from that ion will change the pH of the solution, which can be identified by Ion Torrent's ion sensor. If the nucleotide is not incorporated, no voltage change will be recorded and no base will be called. If there are two identical bases on the DNA strand, the voltage will be double, and the chip will record two identical bases called. Direct identification allows recordation of nucleotide incorporation in seconds. Library preparation for the Ion Torrent platform generally involves ligation of two distinct adaptors at both ends of a DNA fragment.

The DNA sequencing technology utilizes an Illumina sequencing platform, which generally employs cluster amplification of library members onto a flow cell and a sequencing-by-synthesis approach. Cluster-amplified library members are subjected to repeated cycles of polymerase-directed single base extension. Single-base extension can involve incorporation of reversible-terminator dNTPs, each dNTP labeled with a different removable fluorophore. The reversible-terminator dNTPs are generally 3' modified to prevent further extension by the polymerase. After incorporation, the incorporated nucleotide can be identified by fluorescence imaging. Following fluorescence imaging, the fluorophore can be removed and the 3' modification can be removed resulting in a 3' hydroxyl group, thereby allowing another cycle of single base extension. Library preparation for the Illumina platform generally involves ligation of two distinct adaptors at both ends of a DNA fragment.

Therefore, the present invention presents the advantage of being usable with the NGS available on the market. These next-generation sequencing platforms are known from the skilled person in the art and will not be further described herein.

The different steps of the present detection method will be described below.

### Step (a): Shearing of the genomic DNA

### Shearing/Fragmentation (i):

According to one aspect of the invention, the genomic DNA extracted (started DNA) from the biological sample is sheared/ fragmented/sized to the target sequences having a desired length.

The extracted genomic DNA may be sheared by any means known in the art, such as for example, by mechanical shearing, by nebulization, or by sonication.

In particular, three approaches are available to shear/fragment nucleic acid chains: physical, enzymatic, and chemical. DNA fragmentation is typically done by physical methods (i.e., acoustic shearing and sonication) or enzymatic methods (i.e., non-specific endonuclease cocktails and transposase tagmentation reactions).

For instance, acoustic shearing with a Covaris instrument (Covaris, Woburn, MA) is typically done to obtain DNA fragments in the 100-5000 bp range, while Covaris g-TUBEs are employed for the 6-20 Kbp range necessary for mate-pair libraries. Enzymatic methods include digestion by DNase I or Fragmentase, a two enzyme mix (New England Biolabs, Ipswich MA). An alternative enzymatic method for fragmenting DNA is Illumina's Nextera tagmentation technology (Illumina, San Diego, CA) in which a transposase enzyme simultaneously fragments and inserts adapter sequences into dsDNA. This method has several advantages, including reduced sample handling and preparation time.

### Subsequent steps of the pre-library preparation (ii) to (vi):

With the exception of Illumina's Nextera prep, steps before library preparation (b) generally entail: (i) shearing/fragmentation above-mentioned, (ii) end-repair, (iii) phosphorylation of the 5' prime ends, (iv) A-tailing of the 3' ends to facilitate ligation to sequencing adapters, (v) ligation of adapters, and (vi) some number of pre-PCR cycles to enrich for product that has adapters ligated to both ends.

For instance, the pre-PCR (sub-step vi) may be performed by using the following conditions: 1 cycle of 2 min at 98°C, 10 cycles of 30 sec at 98°C, followed by 30 sec at 60°C and 1 min at 72°C. The final elongation is 5 min at 72°C.

The primary differences in an Ion Torrent workflow are the use of blunt-end ligation to different adapter sequences.

In general, once the starting DNA has been sheared, the fragment ends are blunted and 5' phosphorylated using a mixture of three enzymes: T4 polynucleotide kinase, T4 DNA polymerase, and Klenow Large Fragment. Next, the 3' ends are A-tailed using either Taq polymerase or Klenow Fragment (exo-). Taq is more efficient at A-tailing, but Klenow (exo-) can be used for applications where heating is not desired, such as preparing mate-pair libraries. During the adapter ligation reaction the optimal adapter: fragment ratio is -10:1, calculated on the basis of copy number or molarity. Too much adapter favors formation of adapter dimers that can be difficult to separate and dominate in the subsequent PCR amplification. Bead or column-based cleanups can be performed after end repair and A-tail reactions, but after ligation we find bead-based cleanups are more effective at removing excess adapter dimers.

To facilitate multiplexing, different barcoded adapters can be used with each sample. Alternatively, barcodes can be introduced at the PCR amplification step by using different barcoded PCR primers to amplify different samples. High quality reagents with barcoded adapters and PCR primers are readily available in kits from many vendors.

An alternative method is the Nextera DNA Sample Prep Kit (Illumina), which prepares genomic DNA libraries by using a transposase enzyme to simultaneously fragment and tag DNA in a single-tube reaction termed "tagmentation". The engineered enzyme has dual activity; it fragments the DNA and simultaneously adds specific adapters to both ends of the fragments. These adapter sequences are used to amplify the insert DNA by PCR. The PCR reaction also adds index (barcode) sequences. The preparation procedure improves on traditional protocols by combining DNA fragmentation, end-repair, and adaptor-ligation into a single step. This protocol is very sensitive to the amount of DNA input compared with mechanical fragmentation methods. In order to obtain transposition events separated by the appropriate distances, the ratio of transposase complexes to sample DNA is critical. Because the fragment size is also dependent on the reaction efficiency, all reaction parameters, such as temperatures and reaction time, are critical and must be tightly controlled.

Preferably, the sheared genomic DNA obtained at step (a) has a length higher than or equal to 300 bp, preferably ranging from 300 to 600 bp, and ideally ranging from 300 to 350 bp

As used herein, a length higher than or equal to 300 bp includes the following values or any intervals between these values: 300; 310; 320; 330; 340; 350; 360; 370; 380; 390; 400; 410; 420; 430; 440; 450; 460; 470; 480; 490; 500; 510; 520; 530; 540; 550; 560; 570; 580; 590; 600; 610; 620; 630; etc.

### Step (b) Library preparation

According to an embodiment of the invention, the preparation of the library comprises
(b1) the hybridization of the sheared genomic DNA obtained at step (a) with a specific set of capture probes, so as to enrich the sheared genomic DNA contained in the library, and
(b2) the library amplification so as to increase the sheared genomic DNA contained in the library.

As used herein, the terms "library" is used herein and can refer to a plurality of the sheared genomic DNA obtained from a biological sample.

### Step (b1) Hybridization of the sheared genomic DNA obtained at step (a)

This step is performed by using hybrid capture technology.

Especially, in hybrid capture, sheared DNA sequences obtained at step (a) are hybridized to the combination of capture probes according to the invention.

The combination of capture probes comprises at least 43 capture probes having at least 110 nucleotides in length and at least 75 % sequence homology with respectively SEQ ID NO:1 to SEQ ID NO:43, said capture probes being able to hybridize on fragments from at least 5 target regions selected from ALK (NM_004304.4), BRAF (NM_004333.4), EGFR (NM_005228.3), EIF1AY (NM_004681.3), ERBB2 (NM_004448.2), ERBB4 (NM_005235.2), KDR, KIT (NM_000222.2), KRAS(NM_033360.2), MAP2K1 *MAP2K1* (NM_002755.3), MET *MET* (NM_001127500.1), NRAS (NM_002524.4), PDGFRA *PDGFRA* (NM_006206.4), SLC28A1 (NM_004213.4), TP53 (NM_000546.5).

Each of the target regions is herein specified by their 3-letter gene symbol and their NCBI Reference Sequence.

In general, the combination of capture probes comprises further at least 4 additional capture probes comprising at least 110 nucleotides and at least 75 % sequence homology with respectively the probes of SEQ ID NO:44 to SEQ ID NO:47 and/or the probes of SEQ ID NO:48 to SEQ ID NO:51 or combination thereof.

Preferably, the combination of capture probes comprises further at least 100 additional capture probes, preferably at least 200, in particular at least 300, and ideally at least 1178 additional capture probes comprising at least 110 nucleotides and at least 75 % sequence homology with respectively the probes of SEQ ID NO:52 to SEQ ID NO:1229.

In particular, the library preparation at step (b) comprises the implementation of:
- 43 capture probes having at least 75%, especially 80%, preferably 85%, in particular 90% and ideally 95%, and possibly 100% sequence homology with the capture probes of SEQ ID NO:1 to SEQ ID NO:43, respectively and/or;
- additional capture probes having at least 75%, especially 80%, preferably 85%, in particular 90% and ideally 95% and possibly 100% sequence homology with the capture probes of SEQ ID NO:44 to SEQ ID NO:47 and/or;
- additional capture probes having at least 75%, especially 80%, preferably 85%, in particular 90% and ideally 95% and possibly 100% sequence homology with the capture probes with the capture probes of SEQ ID NO:48 to SEQ ID NO:51 and/or;
- additional capture probes having at least 75%, especially 80%, preferably 85%, in particular 90% and ideally 95% and possibly 100% sequence homology with the capture probes with the capture probes of SEQ ID NO:52 to SEQ ID NO:1229, respectively.

In general, the library preparation at step (b) comprises the implementation of capture probes comprising from 110 to 150 nucleotides in length, preferably from 120 to 140 nucleotides in length and ideally from 125 to 135 nucleotides in length from SEQ ID NO: 1 to SEQ ID NO: 1229.

According to an embodiment of the invention, the combination of capture probes according to the invention is biotinylated. The biotin is bound to streptavidin beads and then non-bound DNA is washed away. This has the advantage of more reliable detection of copy number changes.

In general, the library preparation can comprise any method known in the art or as described herein which is suitable with the combination of capture probes according to the invention.

Preferably, the hybridization with said capture probes takes place at a temperature of at least 55°C, preferably at least at 58°C and preferably at a temperature ranging from 60°C to 70°C or from 58 to 65°C.

In general, the sheared genomic DNA captured at step (b) has a length higher than or equal to, 300 bp, preferably ranging from 300 to 600 bp, and ideally ranging from 300 to 350 bp.

After DNA shearing and purification with magnetic beads, sheared genomic DNA is end repaired for instance, thanks to specific polymerase enzyme at 20°C for 30 minutes. Next, DNA sample may be purified with magnetic beads and adenylated at 3' end thanks to a specific enzyme at 37°c for 30 minutes.

Afterwards, once genomic DNA fragments purified with magnetic beads, they may be ligated with adaptor oligo (specific of sequencing technology chosen) thanks to for instance a DNA ligase at 20°C for 15 minutes. Then, DNA fragments are purified for instance with magnetic beads and amplified by PCR with a very high fidelity polymerase (such as : 98°C 2 min, 6 to 10 cycles of 98°C 30 sec + 65°C 30 sec + 72°C 1 min, and 72°C 10 min).

After purification with magnetic beads, the totality of amplified genomic DNA may be hybridized with the probe cocktail (for instance, 95°C 5 min, then 65°C overnight).

Then, hybridized genomic DNA fragments may be captured thanks to streptavidin-coated magnetic beads. Finally, captured libraries may be amplified with indexing primers by PCR with a very high fidelity polymerase (such as: 98°C 2 min, 10 to 16 cycles of 98°C 30 sec + 57°C 30 sec + 72°C 1 min, and 72°C 10 min). Next, DNA sample may be purified with magnetic beads. Quantity and quality of indexed libraries are assessed by Tapestation, Qubit, Bioanalyzer or qPCR.

### Step (b2) Library amplification

Library amplification is advantageously made by PCR technology.

To facilitate multiplexing, different barcoded adapters can be used with each sample. Alternatively, barcodes can be introduced at the PCR amplification step by using different barcoded PCR primers to amplify different samples. High quality reagents with barcoded adapters and PCR primers are readily available in kits from many vendors.

### Step (c) Sequencing

Next, sheared genomic DNA, contained in the library issued at step (b), is sequenced.

In general, the target-enriched libraries are sequenced using any methods known in the art or as described herein in part "Next generation sequencing platforms". Sequencing can reveal the presence of mutations in one or more cancer-related genes in the set.

In some embodiments a subset of 5, 6, 7, 8, 9, 10, 15 genes harboring the mutations are selected for further monitoring by assessment of cell-free DNA in a fluid sample isolated from the subject at later time points.

In some embodiments a subset of no more than 5 genes harboring the mutations are selected for further monitoring by assessment of cell-free DNA in a fluid sample isolated from the subject at later time points.

### Step (d) analyzing of the sequences of said sheared genomic DNA obtained at step (c)

Analyzing step (d) mainly aims at detecting somatic genetic anomalies potentially present in said cancer tissue sample, taken from the subject.

The length of the sequence read can vary depending on the particular sequencing technology utilized. NGS platforms can provide sequence reads that vary in size from tens to hundreds, or thousands of base pairs. In some embodiments of the method described herein, the sequence reads are about 20 bases long, about 25 bases long, about 30 bases long, about 35 bases long, about 40 bases long, about 45 bases long, about 50 bases long, about 55 bases long, about 60 bases long, about 65 bases long, about 70 bases long, about 75 bases long, about 80 bases long, about 85 bases long, about 90 bases long, about 95 bases long, about 100 bases long, about 110 bases long, about 120 bases long, about 130, about 140 bases long, about 150 bases long, about 200 bases long, about 250 bases long, about 300 bases long, about 350 bases long, about 400 bases long, about 450 bases long, about 500 bases long, about 600 bases long, about 700 bases long, about 800 bases long, about 900 bases long, about 1000 bases long, or more than 1000 bases long.

Partial sequencing of DNA fragments present in the sample can be performed, and sequence tags comprising read that map to a known reference genome can be counted. Only sequence reads that uniquely align to the reference genome can be counted as sequence tags. In one embodiment, the reference genome is the human reference genome NCBI38/hg19 sequence, which is available on the world wide web at genome.ucsc.edu/. Other sources of public sequence information include GenBank, dbEST, dbSTS, EMBL (the European Molecular Biology Laboratory), and the DDBJ (the DNA Databank of Japan). The reference genome can also comprise the human reference genome NCBI38/hg19 sequence and an artificial target sequences genome, which includes polymorphic target sequences. In yet another embodiment, the reference genome is an artificial target sequence genome comprising polymorphic target sequences.

Mapping of the sequence tags can be achieved by comparing the sequence of the tag with the sequence of the reference genome to determine the chromosomal origin of the sequenced nucleic acid (e.g. cell free DNA) molecule, and specific genetic sequence information is not needed. A number of computer algorithms are available for aligning sequences, including without limitation BLAST (Altschul et al., 1990), BLITZ (MPsrch) (Sturrock & Collins, 1993), FASTA (Person & Lipman, 1988), BOWTIE (Langmead et al, Genome Biology 10:R25.1-R25.10 [2009]), or ELAND (Illumina, Inc., San Diego, CA, USA). In one embodiment, one end of the clonally expanded copies of the DNA molecule is sequenced and processed by bioinformatic alignment analysis for the Illumina Genome Analyzer, which uses the Efficient Large-Scale Alignment of Nucleotide Databases (ELAND) software. Additional software includes SAMtools {SAMtools, Bioinformatics, 2009, 25(16):2078-9), and the Burroughs-Wheeler block sorting compression procedure which involves block sorting or preprocessing to make compression more efficient.

The sequencing platforms described herein generally comprise a solid support immobilized thereon surface-bound oligonucleotides which allow for the capture and immobilization of sequencing library members to the solid support. Surface bound oligonucleotides generally comprise sequences complementary to the adaptor sequences of the sequencing library.

The analyzing of the sequences, at step (d), can be defined by two parameters: depth and coverage rate.

Depth is the number of reads that include a given nucleotide in the reconstructed sequence (in x). Deep sequencing refers to the general concept of aiming for high number of replicate reads of each region of a sequence.

The coverage rate is the zone covered by at least a read (in %).

Preferably, at the analyzing of the sequences (d), the average depth is of at least 200×, preferably ranging from 200 to 10000×, in particular ranging from 1000× to 1500×, and ideally ranging from 1000× to 1200×.

According to an embodiment, at the analyzing of the sequences (d), the coverage rate is of at least 90% at 200×, preferably ranging from 90% to 99.9% at 500×, in particular ranging from 95% to 99.9% at 500×, and ideally ranging from 98% to 99.9% at 500×.

The sequences of said sheared genomic DNA, obtained at step (c), are analyzed for detecting possible somatic genetic anomalies in said tissue sample.

This analysis can be achieved by well-known bioinformatics technics / pipelines, to identify somatic genetic anomalies (potentially tumorous / cancerous) in said sheared genomic DNA from the library in comparison to known somatic genetic anomalies or to normal genomes. These somatic genetic anomalies can consist in somatic single-base mutations (SSM), somatic insertion/deletion mutations (SIM) and larger structural changes (rearrangements and chromosome segment copy number changes).

### COMBINATION OF PROBES

The present invention concerns also a combination of capture probes, as specified hereabove, i.e. comprising at least 51 capture probes comprising at least 110 nucleotides in length and at least 75 % sequence homology with respectively the probes of SEQ ID NO:1 to SEQ ID NO: 51.

Preferably, the combination of capture probes comprises further at least 100 additional capture probes, preferably at least 200, in particular at least 300, and ideally at least 1178 additional capture probes comprising at least 110 nucleotides and at least 75 % sequence homology with respectively the probes of SEQ ID NO:52 to SEQ ID NO:1229.

Of course, all the characteristics described above for the detection method according to the invention are incorporated herein for the description of the combination of probes.

### KIT OF DETECTION

The present invention also refers to a kit of detection of somatic genetic anomalies potentially present in a tissue sample potentially cancerous taken from a subject, comprising the combination of capture probes as defined above.

Of course, all the characteristics described above for the detection method according to the invention or for the combination of probes are incorporated herein for the description of the detection kit.

In one embodiment, the kit according to the invention comprises further at least: nuclease-free water, 10× End Repair Buffer, dNTP Mix, T4 DNA Polymerase, Klenow DNA Polymerase, T4 Polynucleotide Kinase, 10x Klenow Polymerase Buffer, dATP, Exo(-) Klenow, 5x T4 DNA Ligase Buffer, SureSelect Adaptor Oligo Mix, T4 DNA Ligase, SureSelect Primer, SureSelect Illumina Indexing Pre-Capture PCR Reverse Primer, 5× Herculase II Reaction Buffer, 100 mM dNTP Mix, Herculase II Fusion DNA Polymerase, SureSelect Hyb 1, SureSelect Hyb 2, SureSelect Hyb 3, SureSelect Hyb 4, SureSelect Indexing Block 1, SureSelect Block 2, SureSelect Illumina Indexing Block 3, RNase Block solution, Capture Library, Dynabeads MyOne Streptavidin T1, Agencourt AMPure XP beads, plastic 96-well plates, or combination thereof.

In some embodiments, kits of the invention further include a packaging material. As used herein, the term "packaging material" can refer to a physical structure housing the components of the kit. The packaging material can maintain sterility of the kit components, and can be made of material commonly used for such purposes (e.g., paper, corrugated fiber, glass, plastic, foil, ampules, etc.). Kits can also include a buffering agent, a preservative, or a protein/nucleic acid stabilizing agent.

### DESCRIPTION OF THE FIGURES

The present invention will be hereafter illustrated without being limited thereto by means of the following examples. It will be referred to the following figures in the examples:

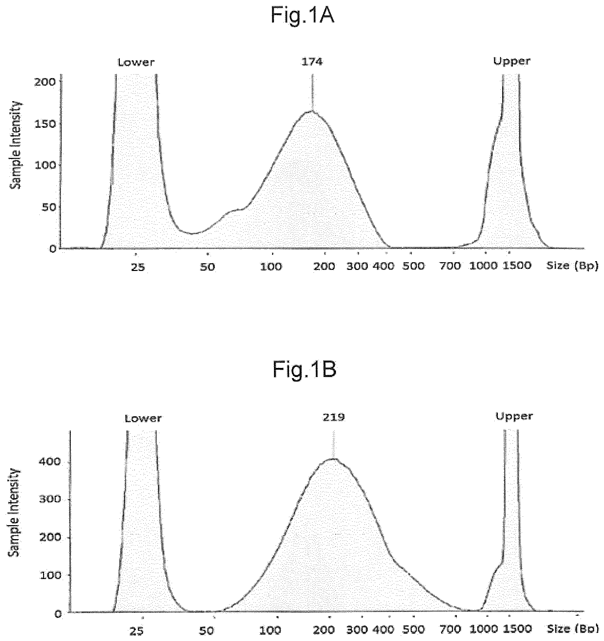

FIG. 1: FIG.1A shows the result obtained with the shearing conditions for the detection kit from FFPE tumor by using the instructions of the manufacturer's protocol and FIG.1B shows the result obtained with the shearing conditions for the detection kit from blood DNA by using the instructions of the manufacturer's protocol;

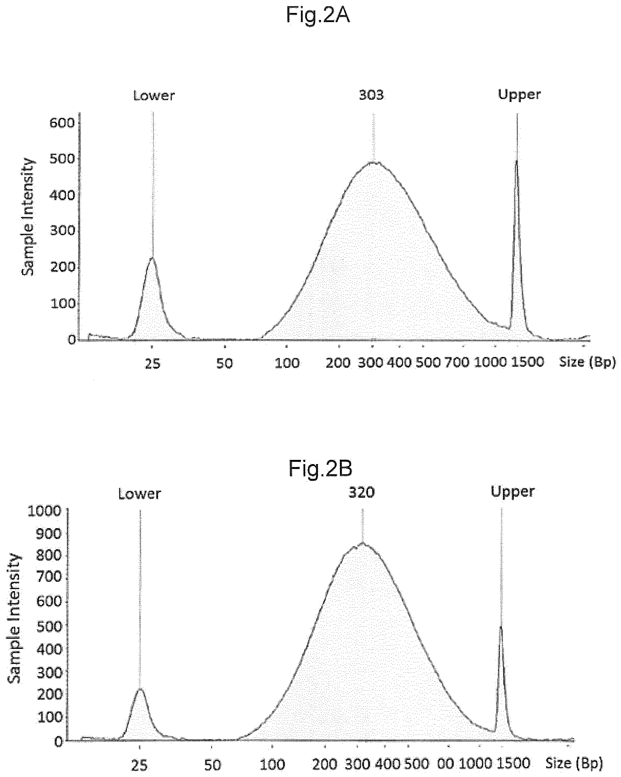

FIG. 2: FIG.2A shows the result obtained with the shearing conditions for the detection kit from FFPE tumor according to the invention by using the protocol described at paragraph 1.c below and FIG.2B shows the result obtained with the shearing conditions for the detection kit from blood DNA according to the invention by using the protocol described at paragraph 1.c below.

### EXAMPLES

### 1- Material and method

### a- Samples

For this assay, 100 samples (tumors, plasmas and commercial samples) were tested. Especially, samples B15 to B17 were obtained from archives of our laboratory, samples CHA, VIA, LOB, RIG, LEM, GAR, TOR, PEN, LUC, LED, POQ, 17BM00026 were obtained from plasma samples obtained in our hospital with STRECK tubes and commercial sample (V600E, G719S and G13D) was obtained from Horizon Discovery.

DNA was extracted from formalin-fixed paraffin-embedded tumors. Tumors were characterized by a pathologist to determine the tumor cell content and sent to the molecular biology platform for DNA extraction. Seven 15 *µ* m tumor slices were extracted using the Maxwell 16 FFPE Plus LEV DNA purification kit (Promega, Madison, USA) according to the manufacturerordingcordingadison, USA) accord assessed by spectrophotometry with absorbance at 230, 260, and 280 nm. DNA was quantified using a fluorimetric assay with a Qubit device.

For plasma samples, 4 ml of plasma obtained from STRECK tubes were extracted with the QlAamp circulating nucleic acid kit (Qiagen, Hilden, Germany), by following the manufacturer's protocol.

### b- Materials/Equipment

For DNA shearing, about 200 ng of DNA were diluted in TE buffer and sonicated thanks to a S220 Covaris Sonicator (Covaris, Woburn, Mass, USA). Libraries were performed with a custom SureSelect XT kit (Agilent). Pre-capture libraries are concentrated with a vacuum concentrator Plus (Eppendorf, Hambourg, Germany). A MixMate device (Eppendorf), and a water bath are also used for capture.

Quality of libraries was assessed thanks to a Tapestation System (Agilent, Santa Clara, CA, USA). Libraries were sequenced on a MiSeq system (Illumina, San Diego, CA, USA) or a NextSeq500 system (Illumina).

Reagents used for the library preparation were Nuclease-free water, 10× End Repair Buffer, dNTP Mix, T4 DNA Polymerase, Klenow DNA Polymerase, T4 Polynucleotide Kinase, 10x Klenow Polymerase Buffer, dATP, Exo(-) Klenow, 5x T4 DNA Ligase Buffer, SureSelect Adaptor Oligo Mix, T4 DNA Ligase, SureSelect Primer, SureSelect Illumina Indexing Pre-Capture PCR Reverse Primer, 5× Herculase II Reaction Buffer, 100 mM dNTP Mix, Herculase II Fusion DNA Polymerase, SureSelect Hyb 1, SureSelect Hyb 2, SureSelect Hyb 3, SureSelect Hyb 4, SureSelect Indexing Block 1, SureSelect Block 2, SureSelect Illumina Indexing Block 3, RNase Block solution. All these reagents were purchased in the SureSelectXT kit. The Custom Capture Library kit is the object of the present invention. Dynabeads MyOne Streptavidin T1, Agencourt AMPure XP beads and plastic 96-well plates were also used for the preparation.

| **Tested probes according to the invention** | SEQ ID NO : 1 - 1229 |
|---|---|
| Probes according to the prior art (comparative examples) | The prior characterization of samples was performed by dedicated custom allelic discrimination kit (Applied Biosystems, Foster City, CA, USA), fragment analysis and Sanger sequencing (Applied Biosystems), TruSeq Custom Amplicon kit (Illumina), Tumor Hotspot MASTR Plus (Multiplicom, Niel, Belgium), or SureSelect XT Human All exon v5 (Agilent) |

### c- Hybrid-capture amplification assay

For these assays, the protocol used is described below. All indicated volumes are for 1 sample (well). These volumes must be adjusted with the number of samples handled at the same time.

### ✔ Assessment of gDNA (genomic DNA) Yield

Quantify freshly extracted gDNA before preparation of the library. Use the fluorometric yield assessment method to quantify intact gDNA (avoid using a spectrophotometer for gDNA yield assessment). Measure the (260/280 nm) ratio and ensure that it is between 1.8 and 2. NOTE: about 200 ng of gDNA are required for the experiment, but a lesser quantity can be used (for example for DNA extracted form plasma)

### ✔ gDNA fragmentation

Dilute about 200 ng DNA in a final volume of 50 µl of TE buffer. Next, sonicate in the Covaris device with the following program: Duty Factor = 10%, Peak Incident Power = 175, Cycles per Burst = 200, bath temperature = 4°C to 8°C, treatment time = 120 sec (for FFPE samples) or 180 sec (for fresh tumor samples) to obtain DNA fragment from 300 to 350 bp.

It is possible to check that your DNA fragments are from 300 to 350 bp thanks to a Bioanalyzer (Agilent) or a Tapestation (Agilent).

### ✔ End Repair

Mix well 35.2µl of Nuclease free water, 10µl 10x End Repair Buffer, 1.6µl dNTP, 1µl T4 DNA Polymerase, 2µl Klenow DNA polymerase, T4 Polynucleotide Kinase, and 50µl of sheared DNA. Incubate 30 min at 20°C and then hold à +4°C.

### ✔ Magnetic purification

Once magnetic beads (AMPure XP) come at room temperature for 30 min, add 180 µl of homogeneous beads to end repaired DNA samples. Mix well and incubate for 5 min at room temperature.

Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 32 µl of nuclease free water to each sample well. After sealing and mixing incubate 2 min at room temperature and replace the plate on the magnetic stand for 3 min. Transfer the supernatant (30µl) containing the purified DNA to a new plate well.

Purified DNA can be stored at -20°C.

### ✔ 3' adenylation

Mix well 11µl of Nuclease free water, 10µl 10x Klenow polymerase Buffer, 1µl dATP, 3µl Exo(-) Klenow, and 30 µl of purified end repaired DNA. Incubate 30 min at 37°C and then hold à +4°C. (if using thermocycler, do not use heated lid).

### ✔ Magnetic purification

Once magnetic beads (AMPure XP) come at room temperature for 30 min, add 90 µl of homogeneous beads to end repaired DNA samples. Mix well and incubates for 5 min at room temperature.

Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 15 µl of nuclease free water to each sample well. After sealing and mixing, incubate 2 min at room temperature and replace the plate on the magnetic stand for 3 min.

Transfer the supernatant (13µl) containing the purified DNA to a new plate well, and proceed immediately the ligation of paired-end adaptor

### ✔ Paired-end adaptor ligation

Mix well 15.5µl of Nuclease free water, 10µl 5x T4 DNA ligase Buffer, 10µl of 1/10^{th} SureSelect Adaptor oligo, 1.5µl T4 DNA Ligase, and 13 µl of purified 3' adenyled DNA. Incubate 15 min at 20°C and then hold à +4°C (if using thermocycler, do not use heated lid).

The plate can be stored at -20°C.

### ✔ Magnetic purification

Once magnetic beads (AMPure XP) come at room temperature for 30 min, add 90 µl of homogeneous beads to end repaired DNA samples. Mix well and incubate for 5 min at room temperature.

Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 32 µl of nuclease free water to each sample well. After sealing and mixing incubate 2 min at room temperature and replace the plate on the magnetic stand for 3 min.

Transfer the supernatant (30µl) containing the purified DNA to a new plate well.

Purified DNA can be stored at -20°C.

### ✔ Amplification of adaptor-ligated library

Mix well 6µl of Nuclease free water, 1.25µl SureSelect primer, 1.25µl SureSelect Illumina indexing Pre-capture PCR reverse primer, 10µl 5x Herculase II Reaction buffer, 0.5µl 100mM dNTP Mix, 1 µl Herculase II Fusion DNA Polymerase and 30 µl of purified adaptor-ligated DNA.

Perform the following PCR program: 98°C for 2 min, 10 cycles of 98°C 30 sec + 65°C 30 sec + 72°C 1 min, 72°C 10 sec and then hold à +4°C.

### ✔ Magnetic purification

Once magnetic beads (AMPure XP) come at room temperature for 30 min, add 90 µl of homogeneous beads to end repaired DNA samples. Mix well and incubate for 5 min at room temperature. Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 30 µl of nuclease free water to each sample well. After sealing and mixing incubate 2 min at room temperature and replace the plate on the magnetic stand for 3 min. Transfer the supernatant (30µl) containing the purified DNA to a new plate well.

Purified DNA can be stored at -20°C.

It is possible to check that your DNA fragments are from 400 to 450 bp thanks to a Bioanalyzer (Agilent) or a Tapestation (Agilent).

### ✔ Hybridization of purified DNA with the capture library

Place the plate containing the purified amplified adaptor-ligated libraries into a vacuum concentrator and dehydrate libraries for 33 min at room temperature (a small volume of liquid, about 3 µl, should be visible).

During this time, prepare hybridization buffer containing: 6.63µl SureSelect Hyb 1, 0.27µl SureSelect Hyb 2, 2.65µl SureSelect Hyb 3, and 3.45µl SureSelect Hyb 4, mix well and keep at room temperature until use.

Next, prepare SureSelect block mix containing: 2.5µl SureSelect indexing block 1, 2.5µl SureSelect block 2, and 0.6µl SureSelect Illumina indexing block 3. Mix well and keep on ice until use.

Add 5.6µl of SureSelect block mix to the well containing DNA and mix by pipetting. Transfer the sealed plate in a thermocycler and incubate at 95°C for 5 min and hold at 65°C for at least 5 min (with the lid at 105°C).

During this time, prepare 5µl of a 10% dilution of RNase block solution on ice, and prepare the capture hybridization mix by mixing well 13µl hybridization buffer mixture prepared above, 5µl of 10% RNase block solution, and 2µl capture library. Keep at room temperature until use.

Once the at least 5 min at 65°C are finished, kept the plate in the thermocycler at 65°C and add 20µl of the capture hybridization mix to each well. Mix well by pipetting and seal the plate.

Incubate the plate overnight at 65°C with a heated lid.

### ✔ Capture of the hybridized DNA

Tomorrow morning, prewarm SureSelect wash buffer 2 at 65°C. Next, completely resuspend Dynabeads MyOne Streptavidin T1 magnetic beads (on vortex). Then, transfer 50µl of these beads into wells of a new PCR plate.

Add 200µl of SureSelect binding buffer to the 50µl beads and mix by pipetting. Then, put the plate on a magnetic stand for 5 min and carefully discard the supernatant without moving the plate from the stand. Wash again 2 more times with SureSelect binding buffer.

Finally, resuspend beads in 200µl SureSelect binding buffer.

By maintaining hybridization plate at 65°C, transfer the entire volume of each hybridization mixture to the plate containing 200µl of washed streptavidin beads, and mix well by pipetting.

Seal the plate and mix vigorously on a MixMate device at 1800 rpm for 30 min at room temperature.

After brief spinning, put the plate on a magnetic stand for 5 min and carefully discard the supernatant without moving the plate from the stand. Remove the plate from the stand and resuspend beads in 200µl of SureSelect wash buffer 1, mix by pipetting. Incubate 15 min at room temperature, briefly spin and put back the plate on a magnetic stand for 5 min. and carefully discard the supernatant without moving the plate from the stand. Remove the plate from the stand, resuspend the beads in 200µl of pre-warm (65°C) wash buffer 2, and mix by pipetting. Seal and incubate the plate in a waterbath at 65°C for 10 min.

Then, put the plate on a magnetic stand for 5 min and carefully discard the supernatant without moving the plate from the stand. Repeat this wash 2 more times.

After the third wash, remove all the supernatant and add 30µl of nuclease-free water to each sample, mix by pipetting, and keep the plate on ice until use.

### ✔ Amplification of the captured libraries

Mix well 18.5µl of Nuclease free water, 5µl 5x Herculase II Reaction buffer, 0.5pl 100mM dNTP Mix, 1 µl Herculase II Fusion DNA Polymerase, 1µl SureSelect Illumina indexing post-capture forward PCR primer and add 31 µl of this preparation to each sample. Next, add 5µl of the appropriate indexing primer to each sample well. Finally, transfer 14µl of the captured DNA into the reaction mix, mix by pipetting, seal the plate and transfer the plate in a thermocycler.

Run the following PCR program: 98°C for 2 min, 16 cycles of 98°C 30 sec + 57°C 30 sec + 72°C 1 min, 72°C 10 min and then hold à +4°C.

### ✔ Magnetic purification

Let magnetic beads (AMPure XP) come to room temperature for 30 min. Next, add 90 µl of homogeneous beads to end repaired DNA samples. Mix well and incubate for 5 min at room temperature. Then, put the plate on a magnetic stand for 5 min and carefully discard the supernatant without moving the plate from the stand.

Still without moving the plate from the stand, wash the beads twice with 200 µl of fresh prepared 70% ethanol for 1 min.

Completely dry the beads (possibly at 37°C for 3 to 5 min), and add 30 µl of nuclease free water to each sample well. After sealing and mixing incubate 2 min at room temperature and replace the plate on the magnetic stand for 3 min. Transfer the supernatant (30µl) containing the purified library to a new plate well.

Purified library can be stored at -20°C for a long time.

### ✔ Quality and quantity assessment of library

Check the quality of library by a fragment analyzer. The library size should be around 500 bp.

Assess the quantity of DNA by using electrophoresis quantification or fluorimetric quantification.

Once library quantified, pool libraries and prepare for injection by following instruction of your sequence device.

### ✔ Sequencing

The sequencing can be performed with 120 to 150 bases for read 1, 9 cycles for index reads, and 120 to 150 bases for read 2.

### 2- Results

### a- Shearing conditions

By referring to Fig.1A to FIG.2B which show the shearing conditions according to the prior art and according to the invention, it can be seen that the shearing conditions according to the invention enables a good fragment pool down and a good quality of sequencing as compared

### b- Sensibility of the detection method of somatic genetic anomalies present in the samples

The results of the sensibility of the combination of capture probes according to the invention are illustrated in the tables below.

| **Origin** | **Sample number** | **Variations observed with classical methods** | **Variations observed with our capture protocol** |
|---|---|---|---|
| Tumor | B08.36 | *EGFR* : Deletion exon 19 | *EGFR* : Deletion exon 19 |
| Tumor | B08.227 | *EGFR :* Deletion exon 19 | *EGFR :* Deletion exon 19 |
| | | *EGFR :* Insertion exon 20 | *EGFR :* Insertion exon 20 |
| Tumor | B09.74 | *EGFR* : L858R | *EGFR* : L858R |
| Tumor | B10.165 | *EGFR* : Deletion exon 19 | *EGFR* : Deletion exon 19 |
| Tumor | B11.437 | *EGFR* : Deletion exon 19 | *EGFR* : Deletion exon 19 |
| Tumor | B12.621 | *EGFR* : Deletion exon 19 | *EGFR* : Deletion exon 19 |
| Tumor | B12.648 | *EGFR* : Deletion exon 19 | *EGFR* : Deletion exon 19 |
| Tumor | B13.425 | *EGFR* : Deletion exon 19 | *EGFR* : Deletion exon 19 |
| Tumor | B13.560 | *EGFR* : Deletion exon 19 | *EGFR :* Deletion exon 19 |
| | | | ***EGFR*** : **S752F** |
| Tumor | B14.488 | *EGFR* : Deletion exon 19 | *EGFR* : Deletion exon 19 |
| Tumor | B14.626 | *EGFR* : L858R | *EGFR* : L858R |
| Tumor | B15.133 | *EGFR* : Deletion exon 19 | *EGFR* : Deletion exon 19 |
| Tumor | B15.169 | *EGFR :* L858R | *EGFR :* L858R |
| | | *EGFR* : T790M | *EGFR* : T790M |
| Tumor | B15.305 | *EGFR* : Deletion exon 19 | *EGFR* : Deletion exon 19 |
| Tumor | B15.812 | *EGFR :* Insertion exon 20 | *EGFR :* Insertion exon 20 |
| | | | ***EGFR*** : **H773Y** |
| Tumor | B15.853 | *EGFR* : Deletion exon 19 | *EGFR* : Deletion exon 19 |
| Tumor | B15.878 | *EGFR* : Deletion exon 19 | *EGFR* : Deletion exon 19 |
| Tumor | B16.237 | *KRAS* : G12V | *KRAS* : G12V |
| Tumor | B16.298 | *EGFR* : Deletion exon 19 | *EGFR* : Deletion exon 19 |
| Tumor | B16.357 | *EGFR* : L858R | *EGFR* : L858R |
| Tumor | B17.12 | *KRAS* : G12S | *KRAS* : G12S |
| Tumor | B16.1251 | *KRAS* : G12V | *KRAS* : G12V |
| Tumor | B17.16 | *KRAS* : G12V | *KRAS* : G12V |
| Tumor | B17.14 | *KRAS* : G12C | *KRAS* : G12C |
| Tumor | B17.21 | *BRAF* : V600E | *BRAF* : V600E |
| Tumor | B17.22 | *EGFR* : Deletion exon 19 | *EGFR* : Deletion exon 19 |
| Tumor | B17.27 | *KIT:* L576P | *KIT:* L576P |
| | | | ***KRAS*: G12D** |
| Tumor | B 17.32 | *KRAS* : G12D | *KRAS* : G12D |
| Tumor | B17.36 | *KRAS* : G12V | *KRAS* : G12V |
| | | | ***EGFR* : H304Y** |
| Tumor | B12.55 | *EGFR* : L858R | *EGFR :* L858R |
| | | | ***EGFR:* D612G** |
| Tumor | B14.415 | *EGFR* : Deletion exon 19 | *EGFR* : Deletion exon 19 |
| Tumor | B14.750 | *EGFR* : L858R | *EGFR* : L858R |
| Tumor | B15.346 | *EGFR* : Deletion exon 19 | *EGFR* : Deletion exon 19 |
| Tumor | B15.849 | *EGFR* : Deletion exon 19 | *EGFR* : Deletion exon 19 |
| Tumor | B16.996 | *KRAS* : G12V | *KRAS* : G12V |
| Tumor | B16.1053 | *KRAS* : Q61H | *KRAS* : Q61H |
| Tumor | B16.1056 | *NRAS* : Q61H | *NRAS* : Q61H |
| Tumor | B16.1078 | *KIT:* V654A | *KIT:* V654A |
| | | *KIT* : Y823D | *KIT* : Y823D |
| Tumor | B16.1120 | *BRAF* : V600E | *BRAF* : V600E |
| Tumor | B16.1124 | *KRAS* : G12C | *KRAS* : G12C |
| Tumor | B16.1126 | *EGFR* : L858R | *EGFR* : L858R |
| Tumor | B16.1127 | *KRAS* : G12C | *KRAS* : G12C |
| Tumor | B16.1129 | *BRAF* : V600E | *BRAF* : V600E |
| Tumor | B16.1130 | *KRAS* : G13D | *KRAS* : G13D |
| Tumor | B16.1137 | *BRAF* : V600E | *BRAF* : V600E |
| Tumor | B16.1138 | *KRAS* : G13D | *KRAS* : G13D |
| Tumor | B16.1148 | *NRAS* : Q61L | *NRAS* : Q61L |
| Tumor | B16.418 | *EGFR* : G719A | *EGFR* : G719A |
| Tumor | B16.430 | *EGFR* : L858R | *EGFR* : L858R |
| Tumor | B16.655 | *EGFR* : G719A | *EGFR* : G719A |
| Tumor | B16.990 | *ERBB2:* insertion exon 20 | *ERBB2* : insertion exon 20 |
| Tumor | B16.501 | *EGFR* : Deletion exon 19 | *EGFR* : Deletion exon 19 |
| Tumor | B16.1141 | *EGFR* : insertion exon 20 | *EGFR* : insertion exon 20 |
| Tumor | B16.1143 | *KRAS* : G12C | *KRAS* : G12C |
| Tumor | B16.1145 | *KRAS* : G12V | *KRAS* : G12V |
| Tumor | B16.1149 | *KRAS* : G13D | *KRAS* : G13D |
| Tumor | B16.1153 | *KRAS* : K117N | *KRAS* : K117N |
| Tumor | B16.1155 | *KRAS* : G13C | *KRAS* : G13C |
| Tumor | B16.1157 | *KRAS* : G12C | *KRAS* : G12C |
| Tumor | B17.41 | *KRAS* : G12A | *KRAS* : G12A |
| Tumor | B17.47 | *EGFR* : L858R | *EGFR* : L858R |
| Tumor | B17.48.1BP | *KRAS* : G13C | *KRAS* : G13C |
| Tumor | B17.48.4DP | *KRAS :* Q61L | *KRAS* : Q61L |
| Tumor | B17.58 | *KRAS* : G12S | *KRAS* : G12S |
| Tumor | B17.59 | *BRAF* : V600E | *BRAF* : V600E |
| Tumor | B17.64 | *KRAS* : G12D | *KRAS* : G12D |
| Tumor | B17. 65 | *BRAF* : V600E | *BRAF* : V600E |
| Tumor | B17.60 | *EGFR* : L858R | *EGFR* : L858R |
| Tumor | B17.61 | *KRAS* : A146P | *KRAS* : A146P |
| Tumor | B17.67 | *BRAF* : V600K | *BRAF* : V600K |
| Tumor | B17.05 | No mutation | No mutation |
| Tumor | B17.11 | No mutation | No mutation |
| Tumor | B17.13 | No mutation | No mutation |
| Tumor | B17.15 | No mutation | No mutation |
| Tumor | B17.19 | No mutation | No mutation |
| Tumor | B17.20 | No mutation | No mutation |
| Tumor | B17.23 | No mutation | No mutation |
| Tumor | B17.05 | No mutation | No mutation |
| Tumor | B17.11 | No mutation | No mutation |
| Tumor | B16.1152 | No mutation | No mutation |
| Tumor | B15.342 | No mutation | ***EGFR :* E709G** |
| | | | ***EGFR** :* **G719C** |
| Tumor | B17.18 | No mutation | ***BRAF* : G596R** |
| Tumor | B15.820 | No mutation | ***EGFR*** : **T751P** |
| Tumor | N16.296 | No mutation | ***EGFR* : S768I** |
| | | | ***EGFR :* V769L** |
| Tumor | B14.632 | No mutation | ***EGFR :* L858R** |
| Plasma | CHA | *KRAS* : G12C | **No mutation** |
| Plasma | VIA | *KRAS* : G12S | *KRAS* : G12S |
| Plasma | LOB | *EGFR* : G719A | *EGFR* : G719A |
| Plasma | RIG | No mutation | No mutation |
| Plasma | LEM | No mutation | No mutation |
| Plasma | GAR | *KRAS :* A146T | *KRAS* : A146T |
| Plasma | TOR | No mutation | No mutation |
| Plasma | PEN | No mutation | No mutation |
| Plasma | LUC | No mutation | No mutation |
| Plasma | LED | *KRAS* : G12C | *KRAS* : G12C |
| Plasma | POQ | *EGFR* : Deletion exon 19 | *EGFR :* Deletion exon 19 |
| | | | ***EGFR* : T790M** |
| Plasma | 17BM00026 | *EGFR* : Deletion exon 19 | *EGFR :* Deletion exon 19 |
| | | | ***EGFR* : T790M** |

| **Origin** | **Expected variations** | **Observed percentage run 1** | **Observed percentage run 2** | **Observed percentage run 3** |
|---|---|---|---|---|
| Commercial | BRAF : V600E | 9% | 9% | 7% |
| | EGFR : G719S | 18% | 20% | 20% |
| | KRAS : G13D | 23% | 26% | 26% |

It could be observed from the above tables that the combination of capture probes according to the invention enables to detect genetic anomalies with a high precision.

## Claims

1. Detection method of somatic genetic anomalies potentially present in a solid tissue sample, potentially cancerous, taken from a subject, wherein said detection method comprises the following steps:
(a) shearing of the genomic DNA extracted from said solid tissue sample, to obtain sheared genomic DNA;
(b) preparing a library by hybridization capture from the sheared genomic DNA obtained at step (a), by the implementation of a combination of at least 51 capture probes having at least 110 nucleotides in length and at least 75% sequence homology with respectively SEQ ID NO:1 to SEQ ID NO:51, the combination of said at least 51 capture probes being able to hybridize on fragments from the following target regions : ALK, BRAF, EGFR, EIF1AY, ERBB2, ERBB4, KDR, KIT, KRAS, MAP2K1, MET, NRAS, PDGFRA, SLC28A1, TP53 ;
(c) sequencing of sheared genomic DNA contained in the library issued at step (b), and
(d) analyzing of the sequences of said sheared genomic DNA obtained at step (c), for detecting somatic genetic anomalies in said tissue sample.

2. Detection method according to claim 1 , wherein the library preparation at step (b) comprises the implementation of the combination of capture probes comprising further at least 100 additional capture probes, preferably at least 200, in particular at least 300, and ideally at least 1178 additional capture probes comprising at least 110 nucleotides in length and at least 75 % sequence homology with SEQ ID NO: 52 to SEQ ID NO: 1229.

3. Detection method according to claim 1 or 2, wherein the library preparation at step (b) comprises the implementation of the combination of capture probes comprising:
- 51 capture probes having at least 75%, preferably 85%, in particular 90% and ideally 95%, sequence homology with the capture probes of SEQ ID NO:1 to SEQ ID NO:51;
- additional capture probes having at least 75%, preferably 85%, in particular 90% and ideally 95% and possibly 100% sequence homology with the capture probes with the capture probes of SEQ ID NO:52 to SEQ ID NO:1229, respectively.

4. Detection method according to any one of the preceding claims, wherein the library preparation at step (b) comprises the implementation of said combination of capture probes comprising from 110 to 150 nucleotides in length, preferably from 120 to 140 nucleotides in length and ideally from 125 to 135 nucleotides in length.

5. Detection method according to any one of the preceding claims, wherein the library preparation at step (b) comprises:
(b1) the hybridization of the sheared genomic DNA obtained at step (a) with the combination of capture probes, so as to enrich the sheared genomic DNA contained in the library,
(b2) the library amplification.

6. Detection method according to claim 5, wherein step (b1) takes place at a temperature of at least 55°C, preferably at least at 58°C and preferably at a temperature ranging from 60°C to 70°C or from 58 to 65°C.

7. Detection method according to any one of the preceding claims, wherein the sheared genomic DNA obtained at step (a) and captured at step (b) have a length higher than or equal to 300 bp, preferably ranging from 300 to 600 pb, and ideally ranging from 300 to 350 pb.

8. Detection method according to any one of the preceding claims, wherein, at the analyzing of the sequences (d), the average depth is of at least 200×, preferably ranging from 200 to 10000×, in particular ranging from 500× to 1500×, and ideally ranging from 500x to 1200×.

9. Detection method according to any one of the preceding claims, wherein, at the analyzing of the sequences (d), the coverage rate is of at least 90% at 200x, preferably ranging from 90% to 99,9% at 500x, in particular ranging from 95% to 99,9% at 500x, and ideally ranging from 98% to 99,9% at 500×.

10. Detection method according to any one of the preceding claims, wherein the somatic genetic anomalies are linked to the cancer selected from at least one of these followings solid cancers: a tumor, polycythemia vera, lymphomas such as Hodgkin's lymphoma, follicular lymphoma or non-Hodgkin's lymphoma, multiple myeloma, Waldenstrom's macroglobulinemia, heavy chain disease, solid tumors, sarcomas, carcinomas such as, e.g., fibrosarcoma, myxosarcoma, liposarcoma, chondrosarcoma, osteogenic sarcoma, lymphangio sarcoma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic, carcinoma, renal cell carcinoma, hepatoma, bile duct carcinoma, choriocarcinoma, seminoma, embryonal carcinoma, Wilms' tumor, cervical cancer, uterine cancer, testicular tumor, lung carcinoma, small cell lung carcinoma, bladder carcinoma, epithelial carcinoma, glioma, craniopharyngioma, ependymoma, pinealoma, hemangioblastoma, acoustic neuroma, oligodendroglioma, meningioma, melanoma, neuroblastoma, retinoblastoma, endometrial cancer, non-small cell lung cancer, glioblastoma or lung cancer.

11. Combination of capture probes, comprising at least 51 capture probes comprising at least 110 nucleotides and at least 75 % sequence homology with respectively the probes of SEQ ID NO:1 to SEQ ID NO:51.

12. Combination of capture probes according to claim 11, comprising further at least:
- 100 additional capture probes, preferably at least 200, in particular at least 300, and ideally at least 1178 additional capture probes comprising at least 110 nucleotides and at least 75 % sequence homology with respectively the probes of SEQ ID NO:52 to SEQ ID NO:1229.

13. Kit of detection of somatic genetic anomalies potentially present in a tissue sample potentially cancerous taken from a subject, comprising the combination of capture probes as defined in claim 11 or claim 12.

## Patentansprüche

1. Nachweisverfahren für somatische genetische Anomalien, die potenziell in einer festen, möglicherweise krebsartigen Gewebeprobe vorliegen, die einem Subjekt entnommen wurde, wobei das Nachweisverfahren die folgenden Schritte umfasst:
(a) Scheren der aus der festen Gewebeprobe extrahierten genomischen DNA, um gescherte genomische DNA zu erhalten;
(b) Erzeugen einer Bibliothek durch Hybridisierungseinfang aus der in Schritt (a) erhaltenen gescherten genomischen DNA durch Implementierung einer Kombination von mindestens 51 Fangsonden mit einer Länge von mindestens 110 Nukleotiden und einer Sequenzhomologie von mindestens 75 %, jeweils mit SEQ ID NO: 1 bis SEQ ID NO: 51, wobei die Kombination der mindestens 51 Fangsonden in der Lage ist, an Fragmente der folgenden Zielregionen zu hybridisieren: ALK, BRAF, EGFR, EIF1AY, ERBB2, ERBB4, KDR, KIT, KRAS, MAP2K1, MET, NRAS, PDGFRA, SLC28A1, TP53;
(c) Sequenzieren der gescherten genomischen DNA, die in der in Schritt (b) ausgegebenen Bibliothek enthalten ist,
und
(d) Analysieren der Sequenzen der in Schritt (c) erhaltenen gescherten genomischen DNA zum Nachweisen somatischer genetischer Anomalien in der Gewebeprobe.

2. Nachweisverfahren nach Anspruch 1, wobei die Erzeugung der Bibliothek in Schritt (b) die Implementierung der Kombination von Fangsonden umfasst, die weitere mindestens 100 zusätzliche Fangsonden umfasst, vorzugsweise mindestens 200, insbesondere mindestens 300 und idealerweise mindestens 1178 zusätzliche Fangsonden mit einer Länge von mindestens 110 Nukleotiden und einer Sequenzhomologie von mindestens 75 % mit SEQ ID NO: 52 bis SEQ ID NO: 1229.

3. Nachweisverfahren nach Anspruch 1 oder 2, wobei die Erzeugung der Bibliothek in Schritt (b) die Implementierung der Kombination von Fangsonden umfasst, umfassend:
• 51 Fangsonden mit mindestens 75 %, vorzugsweise 85 %, insbesondere 90 % und idealerweise 95 % Sequenzhomologie mit den Fangsonden von SEQ ID NO: 1 bis SEQ ID NO: 51;
• zusätzliche Fangsonden mit mindestens 75 %, vorzugsweise 85 %, insbesondere 90 % und idealerweise 95 % und möglicherweise 100 % Sequenzhomologie, jeweils mit den Fangsonden von SEQ ID NO: 52 bis SEQ ID NO: 1229.

4. Nachweisverfahren nach einem der vorhergehenden Ansprüche, wobei die Erzeugung der Bibliothek in Schritt (b) die Implementierung der Kombination von Fangsonden umfasst, umfassend eine Länge von 110 bis 150 Nukleotiden, vorzugsweise eine Länge von 120 bis 140 Nukleotiden und idealerweise eine Länge von 125 bis 135 Nukleotiden.

5. Nachweisverfahren nach einem der vorhergehenden Ansprüche, wobei die Erzeugung der Bibliothek in Schritt (b) umfasst:
(b1) die Hybridisierung der in Schritt (a) erhaltenen gescherten genomischen DNA mit der Kombination von Fangsonden, um die in der Bibliothek enthaltene gescherte genomische DNA anzureichern,
(b2) die Amplifizierung der Bibliothek.

6. Nachweisverfahren nach Anspruch 5, wobei Schritt (b1) bei einer Temperatur von mindestens 55 °C, vorzugsweise mindestens 58 °C und vorzugsweise bei einer Temperatur im Bereich von 60 °C bis 70 °C oder von 58 bis 65 °C stattfindet.

7. Nachweisverfahren nach einem der vorhergehenden Ansprüche, wobei die in Schritt (a) erhaltene und in Schritt (b) eingefangene gescherte genomische DNA eine Länge von mehr als oder gleich 300 bp, vorzugsweise im Bereich von 300 bis 600 bp und idealerweise im Bereich von 300 bis 350 bp aufweist.

8. Nachweisverfahren nach einem der vorhergehenden Ansprüche, wobei bei der Analyse der Sequenzen (d) die durchschnittliche Tiefe mindestens 200x beträgt, vorzugsweise im Bereich von 200x bis 10000x, insbesondere im Bereich von 500x bis 1500x und idealerweise im Bereich von 500x bis 1200x.

9. Nachweisverfahren nach einem der vorhergehenden Ansprüche, wobei bei der Analyse der Sequenzen (d) die Abdeckungsrate bei 200x mindestens 90 % beträgt, vorzugsweise im Bereich von 90 % bis 99,9 % bei 500x, insbesondere im Bereich von 95 % bis 99,9 % bei 500x und idealerweise im Bereich von 98 % bis 99,9 % bei 500x.

10. Nachweisverfahren nach einem der vorhergehenden Ansprüche, wobei die somatischen genetischen Anomalien mit dem Krebs verknüpft sind, der ausgewählt ist aus mindestens einer der folgenden soliden Krebsarten: Tumor, Polycythaemia vera, Lymphome, wie etwa Hodgkin-Lymphom, follikuläres Lymphom oder Non-Hodgkin-Lymphom, multiples Myelom, Waldenström-Makroglobulinämie, Schwerketten-Krankheit, solide Tumoren, Sarkome, Karzinome wie z. B. Fibrosarkom, Myxosarkom, Liposarkom, Chondrosarkom, osteogenes Sarkom, Lymphangiosarkom, Mesotheliom, Ewing-Tumor, Leiomyosarkom, Rhabdomyosarkom, Dickdarmkarzinom, Darmkrebs, Bauchspeicheldrüsenkrebs, Brustkrebs, Eierstockkrebs, Prostatakrebs, Plattenepithelkarzinom, Basalzellkarzinom, Adenokarzinom, Schweißdrüsenkarzinom, Talgdrüsenkarzinom, papilläres Karzinom, papilläre Adenokarzinome, Zystadenokarzinom, Markkarzinom, bronchogenes Karzinom, Nierenzellkarzinom, Hepatom, Gallengangskarzinom, Choriokarzinom, Seminom, embryonales Karzinom, Wilms-Tumor, Gebärmutterhalskrebs, Gebärmutterkrebs, Hodentumor, Lungenkarzinom, kleinzelligem Lungenkarzinom, Blasenkarzinom, Epithelkarzinom, Gliom, Kraniopharyngeom, Ependymom, Pinealom, Hämangioblastom, Akustikusneurinom, Oligodendrogliom, Meningeom, Melanom, Neuroblastom, Retinoblastom, Endometriumkrebs, nichtkleinzelligem Lungenkrebs, Glioblastom oder Lungenkrebs.

11. Kombination von Fangsonden, umfassend mindestens 51 Fangsonden mit mindestens 110 Nukleotiden und mindestens 75 % Sequenzhomologie, jeweils mit den Sonden von SEQ ID NO: 1 bis SEQ ID NO: 51.

12. Kombination von Fangsonden nach Anspruch 11, ferner umfassend mindestens:
- 100 zusätzliche Fangsonden, vorzugsweise mindestens 200, insbesondere mindestens 300 und idealerweise mindestens 1178 zusätzliche Fangsonden, umfassend mindestens 110 Nukleotide und mindestens 75 % Sequenzhomologie, jeweils mit den Sonden von SEQ ID NO: 52 bis SEQ ID NO: 1229.

13. Kit zum Nachweis somatischer genetischer Anomalien, die möglicherweise in einer möglicherweise krebsartigen Gewebeprobe eines Subjekts vorliegen, umfassend die Kombination von Fangsonden gemäß Anspruch 11 oder Anspruch 12.

## Revendications

1. Procédé de détection d'anomalies génétiques somatiques potentiellement présentes dans un échantillon de tissu solide, potentiellement cancéreux, prélevé chez un sujet, ledit procédé de détection comprenant les étapes suivantes :
(a) le cisaillement de l'ADN génomique extrait à partir dudit échantillon de tissu solide afin d'obtenir de l'ADN génomique cisaillé ;
(b) la préparation d'une bibliothèque par capture d'hybridation à partir de l'ADN génomique cisaillé obtenu à l'étape (a), par la mise en oeuvre d'une combinaison d'au moins 51 sondes de capture ayant au moins 110 nucléotides de long et une homologie de séquence d'au moins 75% avec respectivement SEQ ID NO:1 à SEQ ID NO:51, la combinaison desdites au moins 51 sondes de capture étant capable de s'hybrider sur des fragments provenant des régions cibles suivantes : ALK, BRAF, EGFR, EIF1AY, ERBB2, ERBB4, KDR, KIT, KRAS, MAP2K1, MET, NRAS, PDGFRA, SLC28A1, TP53 ;
(c) le séquençage de l'ADN génomique cisaillé contenu dans la bibliothèque établie à l'étape (b), et
(d) l'analyse des séquences dudit ADN génomique cisaillé obtenu à l'étape (c), pour détecter des anomalies génétiques somatiques dans ledit échantillon de tissu.

2. Procédé de détection selon la revendication 1, dans lequel la préparation de la bibliothèque de l'étape (b) comprend la mise en oeuvre de la combinaison de sondes de capture comprenant en outre au moins 100 sondes de capture supplémentaires, de préférence, au moins 200, en particulier, au moins 300, et idéalement au moins 1178 sondes de capture supplémentaires comprenant au moins 110 nucléotides de long et une homologie de séquence d'au moins 75 % avec SEQ ID NO: 52 à SEQ ID NO: 1229.

3. Procédé de détection selon la revendication 1 ou 2, dans lequel la préparation de la bibliothèque de l'étape (b) comprend la mise en oeuvre de la combinaison de sondes de capture comprenant :
- 51 sondes de capture ayant une homologie de séquence d'au moins 75%, de préférence 85%, en particulier 90% et idéalement 95%, avec les sondes de capture de SEQ ID NO:1 à SEQ ID NO:51 ;
- des sondes de capture supplémentaires ayant une homologie de séquence d'au moins 75%, de préférence 85%, en particulier 90% et idéalement 95% et éventuellement 100%, avec respectivement les sondes de capture de SEQ ID NO: 52 à SEQ ID NO: 1229.

4. Procédé de détection selon l'une quelconque des revendications précédentes, dans lequel la préparation de la bibliothèque de l'étape (b) comprend la mise en oeuvre de ladite combinaison de sondes de capture comprenant de 110 à 150 nucléotides de long, de préférence, de 120 à 140 nucléotides de long et idéalement, de 125 à 135 nucléotides de long.

5. Procédé de détection selon l'une quelconque des revendications précédentes, dans lequel la préparation de la bibliothèque de l'étape (b) comprend :
(b1) l'hybridation de l'ADN génomique cisaillé obtenu à l'étape (a) avec la combinaison de sondes de capture, de manière à enrichir l'ADN génomique cisaillé contenu dans la bibliothèque,
(b2) l'amplification de la bibliothèque.

6. Procédé de détection selon la revendication 5, dans lequel l'étape (b1) a lieu à une température d'au moins 55°C, de préférence, au moins 58°C et de préférence, à une température allant de 60°C à 70°C ou de 58 à 65°C.

7. Procédé de détection selon l'une quelconque des revendications précédentes, dans lequel les ADN génomiques cisaillés obtenus à l'étape (a) et capturés à l'étape (b) ont une longueur supérieure ou égale à 300 pb, de préférence allant de 300 à 600 pb, et idéalement allant de 300 à 350 pb.

8. Procédé de détection selon l'une quelconque des revendications précédentes, dans lequel, lors de l'analyse des séquences (d), la profondeur moyenne est d'au moins 200x, de préférence, va de 200 à 10000x, en particulier, va de 500x à 1500x, et idéalement va de 500x à 1200x.

9. Procédé de détection selon l'une quelconque des revendications précédentes, dans lequel, lors de l'analyse des séquences (d), le taux de couverture est d'au moins 90% à 200x, de préférence, va de 90% à 99,9% à 500x, en particulier va de 95% à 99,9% à 500x, et idéalement, va de 98% à 99,9% à 500x.

10. Procédé de détection selon l'une quelconque des revendications précédentes, dans lequel les anomalies génétiques somatiques sont liées au cancer choisi parmi au moins l'un des cancres solides suivants : une tumeur, la polycythémie vraie, les lymphomes tels que le lymphome de Hodgkin, le lymphome folliculaire ou le lymphome non hodgkinien, le myélome multiple, la macroglobulinémie de Waldenström, la maladie des chaînes lourdes, les tumeurs solides, les sarcomes, les carcinomes comme par exemple, le fibrosarcome, le myxosarcome, le liposarcome, le chondrosarcome, l'ostéosarcome, le lymphangio-sarcome, le mésothéliome, la tumeur d'Ewing, le léiomyosarcome, le rhabdomyosarcome, le carcinome du côlon, le cancer colorectal, le cancer du pancréas, le cancer du sein, le cancer des ovaires, le cancer de la prostate, le carcinome épidermoïde, le carcinome basocellulaire, l'adénocarcinome, le carcinome des glandes sudoripares, le carcinome des glandes sébacées, le carcinome papillaire, les adénocarcinomes papillaires, le cystadénocarcinome, le carcinome médullaire, le carcinome bronchogénique, l'hypeméphrome, l'hépatome, le carcinome des voies biliaires, le choriocarcinome, le séminome, le carcinome embryonnaire, la tumeur de Wilm, le cancer du col de l'utérus, le cancer de l'utérus, la tumeur testiculaire, le carcinome pulmonaire, le cancer pulmonaire à petites cellules, le cancer de la vessie, le carcinome épithélial, le gliome, le craniopharyngiome, l'épendymome, le pinéalome, l'hémangioblastome, le neurinome de l'acoustique, l'oligodendrogliome, le méningiome, le mélanome, le neuroblastome, le rétinoblastome, le cancer de l'endomètre, le cancer du poumon non à petites cellules, le glioblastome ou le cancer du poumon.

11. Combinaison de sondes de capture, comprenant au moins 51 sondes de capture comprenant au moins 110 nucléotides et une homologie de séquence d'au moins 75% avec respectivement les sondes de SEQ ID NO:1 à SEQ ID NO:51.

12. Combinaison de sondes de capture selon la revendication 11, comprenant en outre au moins :
- 100 sondes de capture supplémentaires, de préférence au moins 200, en particulier au moins 300, et idéalement au moins 1178 sondes de capture supplémentaires comprenant au moins 110 nucléotides et une homologie de séquence d'au moins 75% avec respectivement les sondes de SEQ ID NO:52 à SEQ ID NO:1229.

13. Kit de détection d'anomalies génétiques somatiques potentiellement présentes dans un échantillon de tissu potentiellement cancéreux prélevé chez un sujet, comprenant la combinaison de sondes de capture telle que définie dans la revendication 11 ou la revendication 12.
